# EUROPÄISCHE PATENTANMELDUNG

(11) **EP 2 194 086 A1**
(43) Veröffentlichungstag der Anmeldung: **09.06.2010**
(21) Anmeldenummer: 09175185.9
(22) Anmeldetag: 06.11.2009
(51) Int. Cl.: C08G 77/18, C08G 77/46, C08L 83/12, C09D 183/12, A61K 8/894, C04B 41/49

(54) **Verfahren zur Modifizierung von Oberflächen**

(30) Priorität: 05.12.2008 DE 102008044373; 25.05.2009 DE 102009022628
(71) Anmelder: Evonik Goldschmidt GmbH, 45127 Essen (DE)
(72) Erfinder: Ebbrecht, Thomas, 58300, Wetter / Ruhr (DE); Schubert, Frank, 47506, Neukirchen-Vluyn (DE); Naumann, Matthias, Greensboro, NC 27407 (US); Knott, Wilfried, 45355, Essen (DE); Lehmann, Kathrin, 51377, Leverkusen (DE); Venzmer, Joachim, 45239, Essen (DE); De Gans, Berend-Jan, 45478, Mülheim an der Ruhr (DE); Silber, Stefan, 47804, Krefeld (DE); Henning, Frauke, 45130, Essen (DE)

(57) **Zusammenfassung**

Verwendung von einem oder mehreren gemischten härtbaren hydroxygruppenhaltigen Silylpolyether als Bestandteil von Zusammensetzungen, als Modifizierungsmittel für Oberflächen, wobei die Silylpolyether durch DMCkatalysierte Alkoxylierung von epoxyfunktionellen Alkoxysilanen hergestellt werden.

## Beschreibung

Die Erfindung betrifft ein Verfahren zur Modifizierung von flächigen und Partikeloberflächen durch Silylgruppen tragende Hydroxylverbindungen enthaltende härtbare Zusammensetzungen.

Als Silylgruppen tragende Hydroxylverbindungen, die im Sinne dieser Erfindung Verwendung finden, werden alle Reaktionsprodukte verstanden, die durch Alkoxylierung von epoxyfunktionellen Silanen an Doppelmetallcyanid-Katalysatoren gemäß dem in der noch nicht vorveröffentlichten Schrift DE 10 2008 000360.3 beschriebenen Verfahren hergestellt werden können; insbesondere können diese Verbindungen auch Siloxangruppen tragen. Diese Produkte werden im Weiteren als Silylpolyether 1 bezeichnet. Eine Silylgruppe im Rahmen dieser Erfindung ist durch unterschiedliche oder gleiche organische oder oxyorganische Reste gekennzeichnet.

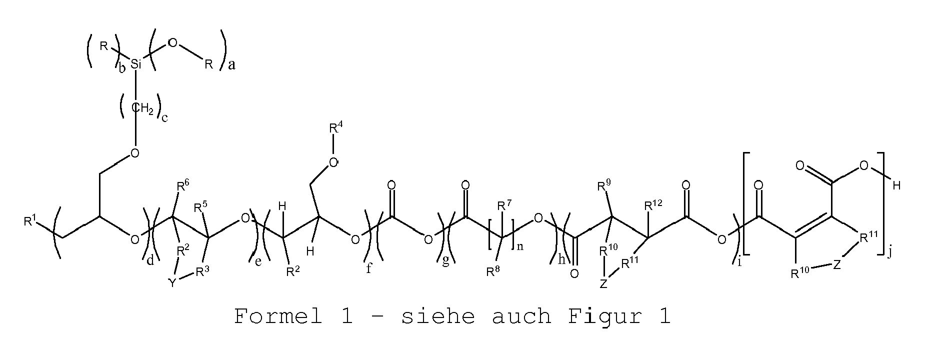

Im Rahmen dieser Erfindung umfasst der Begriff Polyether sowohl Polyether, Polyetherole, Polyetheralkohole, Polyetherester aber auch Polyethercarbonate, die gegebenenfalls synonym zueinander verwendet werden. Dabei ist nicht erforderlich, dass der Ausdruck "Poly" damit einhergehen muss, dass es sich um eine Vielzahl von Etherfunktionalitäten oder Alkoholfunktionalitäten im Molekül oder Polymer handelt. Vielmehr wird dadurch nur angedeutet, dass zumindest Wiederholungseinheiten einzelner Monomerbausteine oder aber Zusammensetzungen vorliegen, die eine höhere Molmasse und zudem auch noch eine gewisse Polydispersität aufweisen.
Das Wortfragment "Poly" umfasst im Zusammenhang mit dieser Erfindung nicht nur ausschließlich Verbindungen mit zumindest 3 Wiederholungseinheiten eines oder mehrerer Monomere im Molekül, sondern insbesondere auch solche Zusammensetzungen von Verbindungen, die eine Molekulargewichtsverteilung aufweisen und dabei ein mittleres Molekulargewicht von mindestens 200 g/mol besitzen. Bei dieser Definition ist dem Umstand Rechnung getragen, dass es auf dem betrachteten Gebiet der Technik üblich ist, solche Verbindungen bereits als Polymere zu bezeichnen, auch wenn sie nicht einer Polymerdefinition analog OECD- oder REACH-Richtlinien zu genügen scheinen.
Unter Modifizierung wird hierbei die chemische oder physikalische Anbindung eines Modifizierungsmittels an die jeweilige feste Oberfläche verstanden. Die Modifizierung kann dabei bezogen auf die feste Oberfläche wahlweise partiell oder vollständig sein, wobei die Feststoffoberfläche mit einer geschlossenen Monoschicht oder auch mit einer Multischicht belegt sein kann. Eine Modifizierung im Sinne dieser Definition schließt auch Oberflächenbeschichtungen mit ein, welche in der Regel vollflächig sind, wie z.B. bei Lacken und Farben.
Modifizierungen von Flächen oder Partikeln zur Modifikation von Oberflächen werden aus vielfältigen Gründen und nach unterschiedlichsten Verfahren durchgeführt und sind dem Fachmann aus der Literatur bekannt. Modifizierungen dienen im Allgemeinen dazu, die chemischen und physikalischen Eigenschaften von Oberflächen eines Trägermaterials durch Aufbringen einer meist dünnen Schicht eines Modifizierungsmittels zielgerichtet auf den jeweils gewünschten Anwendungszweck zu modifizieren. Zum Beispiel erfüllen Modifizierungsmittel wichtige Funktionen als Haftvermittler, Primer, Lacke, Hydrophobierungsmittel, oder Netzmittel. Allen Varianten der Beschichtungen gemeinsam ist die Aufbringung einer möglichst gut auf dem jeweiligen Untergrund haftenden Schicht durch Auftragen eines oftmals flüssigen oder pulverförmigen, leicht zu applizierenden Modifizierungsmittels. Je nach Art der zu modifizierenden Oberfläche, der chemisch-physikalischen Natur des jeweiligen Modifizierungsmittels und dem anwendungstechnischen Ziel der angestrebten Oberflächenmodifizierung kommen ganz unterschiedliche Verfahren der Schichtaufbringung zur Anwendung. Neben zum Beispiel thermischen und elektrochemischen Verfahren spielen insbesondere chemische Modifizierungsverfahren eine herausragende Rolle.
Ebenso vielfältig sind die der Oberflächenmodifizierung zugänglichen Substrate. Neben eher flächigen Trägermaterialien wie Metallen, Beton, Kunststoffen, Holz oder Glas sind es feste Partikel, Fasern und beispielsweise textile Gewebe, die durch Applizieren dünner Schichten oberflächenmodifiziert werden.
So ist es Stand der Technik, Oberflächen anorganischer Partikel, makroskopischer anorganischer Oberflächen und anorganischer Fasern, wie z.B. solche der Metalloxide, Mischoxide, Nitride, Hydroxide, Carbide, Carbonate, Silikate, Pigmente, Ruße, Elemente oder Legierungen zu modifizieren. Ebenso dem Fachmann bekannt ist es, organische Partikel, makroskopische organische Oberflächen und Fasern, wie z. B. solche aus Siliconharzen, organomodifizierten Siliconen, organischen Polymeren oder Biopolymeren zu modifizieren.

Besonders vorteilhaft kann es sein, wenn das eingesetzte Modifizierungsmittel mindestens eine funktionelle Gruppe aufweist, die mit der zu modifizierenden Oberfläche kovalente, ionische oder koordinative Bindungen oder Wasserstoffbrückenbindungen eingehen kann. Es kann sich bei diesen funktionellen Gruppen beispielsweise um Carbonsäuregruppen, Säurechloridgruppen, Estergruppen, Nitril- und Isonitrilgruppen, OH-Gruppen, SH-Gruppen, Epoxidgruppen, Anhydridgruppen, Säureamidgruppen, primäre, sekundäre und tertiäre Aminogruppen, SiOH-Gruppen, hydrolysierbare Alkoxysilane oder CH-acide Gruppierungen wie z. B. in β-Dicarbonylverbindungen, beispielsweise Acetylaceton, 2,4-Hexandion, 3,5-Heptandion, Diacetyl oder Acetessigsäure, handeln. Ebenso können auch mehr als eine derartige Gruppe in dem Modifizierungsmittel vorhanden sein, wie z. B. in Betainen, Aminosäuren, beispielsweise Glycin, Alanin, β-Alanin, Valin, Leucin, Isoleucin, Arginin und Aminocapronsäure, sowie in EDTA. Carbonsäuren zur Oberflächenmodifizierung sind beispielsweise Fettsäuren, Ameisensäure, Essigsäure, Propionsäure, Buttersäure, Pentansäuren, Hexansäure, (Meth)Acrylsäure, Adipinsäure, Bernsteinsäure, Fumarsäure, Itaconsäure, Stearinsäure, Hydroxystearinsäure, Ricinolsäure und Polyethercarbonsäuren und deren entsprechende Stereoisomere, Anhydride, Chloride, Ester und/oder Amide, beispielsweise M ethoxyessigsäure, 3,6-Dioxaheptansäure und 3,6,9-Trioxadecansäure sowie die entsprechenden Säurechloride, -ester und -amide oder auch kationische Gruppen tragende Modifizierungsmittel.

Von besonderem Vorteil ist es, wenn gleichzeitig auch auf der Oberfläche des zu modifizierenden Substrats funktionelle Gruppen wie Hydroxylgruppen, SH-Gruppen, Aminogruppen oder beispielsweise Carbonsäuregruppen vorhanden sind, so dass sich zwischen Trägermaterial und Modifzierungsmittel intensive physikalische Wechselwirkungen ausbilden können oder es zu chemischen Reaktionen zwischen reaktiven funktionellen Gruppen des **Modifizierungsmittels mit denen auf der** Substratoberfläche kommt. Auf diesem Wege wird das Modifizierungsmittel dauerhaft auf dem jeweiligen Untergrund verankert. Insbesondere die chemische Anbindung des Modifizierungsmittels an das Trägermaterial ist eine effiziente und in der Praxis weit verbreitete Methode, um ein späteres Ablösen der Beschichtung z.B. unter dem Einfluss von Wasser oder Lösemitteln zu vermeiden und eine permanente, sich auch über einen längeren Zeitraum nicht verändernde Oberflächenqualität zu gewährleisten.

Die Art der physikalisch oder chemisch wirksamen Ankergruppe im Modifizierungsmittel ist dabei in jedem Fall exakt auf die Beschaffenheit und den Typ der reaktiven funktionellen Gruppen der Substratoberfläche abzustimmen. Wichtige weitere Auswahlkriterien für die Selektion des am besten geeigneten Modifizierungsmittels sind der pH-Wert, Feuchtigkeitsgehalt oder die Porosität des jeweiligen Untergrunds.

Daher gibt es kein universell für alle Typen von Oberflächen einsetzbares chemisches System zur Oberflächenmodifizierung, sondern eine Vielzahl von meist reaktive Gruppen tragenden Verbindungsklassen, die zur Oberflächenmodifikation herangezogen werden. Der Stand der Technik listet zum Beispiel reaktive monomere und polymere Isocyanat-, Epoxid-, Acrylat-, Silan-, Carboxylat-, Amin-, Hydroxyl- und Mercapto-Funktionen aufweisende Verbindungen, die je nach Substrat auf unterschiedlichem chemischen/physikalischen Wege auf dem Untergrund fixiert werden.

Neben der zur Anbindung an das Substrat befähigten funktionellen Gruppe kann das Modifizierungsmittel weitere Reste aufweisen, welche die Oberflächeneigenschaften des Substrats modifizieren helfen. Solche Reste, oder auch Teile davon, können beispielsweise hydrophob oder hydrophil sein oder eine oder mehrere funktionelle Gruppen tragen, um auf diese Weise Feststoffpartikel oder auch Fasern kompatibel zum umgebenden Medium zu machen, sie zu inertisieren oder reaktionsfähig gegenüber z.B. weiteren anzubindenden Materialien zu machen, was auch eine Anbindung an die umgebende Matrix mit einschließt. Diese funktionellen Gruppen können beispielsweise ausgewählt sein aus dem Bereich der Alkyl-, Aryl-, Alkaryl-, Aralkyl-, Fluoralkyl-, Hydroxy-, Alkoxy-, Polyalkoxy-, Epoxy-, Acryloxy-, Methacryloxy-, Acrylat-, Methacrylat-, Carboxy- Amino-, Sulfonyl-, Sulfat-, Phosphat-, Polyphosphat-, Phosphonat, Amid-, Sulfid-, Hydrogensulfid-, Halogenalkyl-, Halogenaryl-, Acyl- und ionische, beispielsweise anionische, kationische oder amphotere Gruppen.
Zu den am weitesten verbreiteten Modifizierungsmitteln gehören hydrolysierend vernetzbare Silane der Formel (2),

AₓSiB₍₄₋ₓ₎ (2)

wobei A gleiche oder verschiedene nicht hydrolysierbare Gruppen, B = gleiche oder verschiedene hydrolysierbare Gruppen oder Hydroxygruppen und x = 1, 2, 3 oder 4 darstellt.

In der allgemeinen Formel (2) können die hydrolysierbaren Gruppen B beispielsweise H, Halogen-, Alkoxy-, bevorzugt Methoxy-, Ethoxy-, i-Propoxy-, n-Propoxy- oder Butoxy-, Aryloxy-, (bevorzugt Phenoxy-, Acyloxy-, bevorzugt Acetoxy- oder Propionyloxy-, Acyl-, bevorzugt Acetyl-, Amino-, Monoalkylamino- oder Dialkylamino-Gruppen sein. Der nicht hydrolysierbare Rest A kann beispielsweise ein Alkyl-, Alkenyl-, Alkinyl-, Aryl-, Alkylaryl- oder Aralkyl-Rest sein. Die Alkylkette kann 0 bis 50, vorzugsweise 0 bis 22 Kohlenstoffatome aufweisen und auch durch Heteroatome wie Sauerstoff oder Stickstoff oder Schwefel unterbrochen sein oder auch ein Siliconrest sein. Der aromatische Rest kann auch heteroaromatisch sein. Die Reste A und B können ggf. einen oder mehrere übliche Substituenten, wie beispielsweise Halogen oder Alkoxy aufweisen.

Nicht hydrolysierbare Reste A nach der Formel (2) mit funktionellen Gruppen können ausgewählt werden aus dem Bereich der Glycidyl- oder Glycidyloxyalkylen-Reste, wie beispielsweise β-Glycidyloxyethyl, γ-Glycidyloxypropyl, δ-Glycidyloxypropyl, ε-Glycidyloxypentyl, ω-Glycidyloxyhexyl oder 2-(3,4-Epoxycyclohexyl)ethyl, der Methacryloxyalkylen- und Acryloxyalkylen-Reste, wie beispielsweise Methacryloxymethyl, Acryloxymethyl, Methacryloxyethyl, Acryloxyethyl, Methacryloxypropyl, Acryloxypropyl, Methacryloxybutyl oder Acryloxybutyl, und dem 3-Isocyanatopropyl-Rest, und/oder cyclische und/oder lineare (poly) urethangruppen-haltige und/oder harnstoffhaltige und/oder (poly)amingruppenhaltiger Rest.

Besonders verbreitet ist der Einsatz von niedrig viskosen, monomeren Trimethoxysilyl- und Triethoxysilylgruppen tragenden Verbindungen, die in Gegenwart von Luftfeuchtigkeit und geeigneten Katalysatoren zumeist schon bei Raumtemperatur in der Lage sind, unter Abspaltung der Alkoxygruppen und Ausbildung von Si-O-Si-Bindungen miteinander zu kondensieren. Solche organofunktionellen monomeren Silane sind zum Beispiel N-Cyclohexylaminomethyltrimethoxysilan, N-Cyclohexyl-3-aminopropyltriethoxysilan, 3-Aminopropyltrimethoxysilan, Vinyltrimethoxysilan, Vinyltriethoxysilan, Vinyldimethoxymethylsilan, 3-Isocyanatopropyltrimethoxysilan, 3-Glycidyloxypropyltrimethoxysilan, 3-Glycidyloxypropyltriethoxysilan, 3-Methacryloxypropyltrimethoxysilan, Methyltrimethoxysilan, Methyltriethoxysilan, Dimethyldimethoxysilan, Phenyltriethoxysilan und Hexadecyltrimethoxysilan. Einen Einstieg in diese Thematik bietet "Silylated Surfaces", edited by Donald E. Leyden and Ward T. Collins, Gordon and Breach Science Publishers, Inc., 1980, ISBN 0-677-13370-7.

Neben monomeren Organosilanen sind Präpolymersysteme und Sol-Gel-Materialien (s. dazu auch "Sol-Gel-Science: The Physics and Chemistry of Sol-Gel Processing", C. Jeffrey Brinker, George W. Scherer, Academic Press Inc., 1990, ISBN 0-12-134970-5), sowie deren Mischungen untereinander, die über reaktive Alkoxysilylgruppen verfügen und vielfach zur Herstellung von Beschichtungen sowie elastischen Dicht- und Klebstoffen im Industrie- und Baubereich verwendet werden, bekannt. So sind alkoxysilanfunktionelle Polyurethane, die über eine Silanpolykondensation vernetzen, lange bekannt. Ein Übersichtsartikel zu dieser Thematik findet sich z.B. in "Adhesives Age" 4/1995, Seite 30 ff. (Autoren: Ta-Min Feng, B. A. Waldmann). Derartige Alkoxysilan-terminierte, feuchtigkeitshärtende Einkomponenten-Polyurethane werden in zunehmendem Maße als weichelastische Beschichtungs-, Dichtungs- und Klebemassen im Bauwesen und in der Automobilindustrie verwendet.
Solche alkoxysilanfunktionellen Polyurethane können gemäß US 3,627,722 oder US 3,632,557 hergestellt werden, indem z.B. Polyetherpolyole mit einem Überschuss Polyisocyanat zu einem NCO-haltigen Präpolymer umgesetzt werden, das dann wiederum mit einem aminofunktionellen Alkoxysilan weiter umgesetzt wird. Das entstehende alkoxysilanfunktionelle Präpolymer enthält Harnstoff und Urethangruppen in hoher Konzentration, die zu einer hohen Viskosität der Produkte führen. Die WO 2007/025667 beschreibt weiterhin modifizierte Alkoxysilangruppen aufweisende Polyurethanpräpolymere, die eine reduzierte Viskosität aufweisen sollen.

Bei einem besonders verbreiteten Typ von alkoxysilanfunktionellen Präpolymeren handelt es sich um alkoxysilanterminierte Präpolymere. Diese können aus unterschiedlichen Bausteinen aufgebaut sein. Üblicherweise besitzen diese Präpolymere ein organisches Rückgrat, d.h. sie sind beispielsweise aus Polyurethanen, Polyethern, Polyestern, Polyacrylaten, Polyvinylestern, Ethylen-Olefincopolymeren, Styrol-Butadiencopolymeren oder Polyolefinen aufgebaut, beschrieben u.a. in EP 0 372 561, WO 00/37533 oder US 6,207,766. Daneben sind aber auch Systeme weit verbreitet, deren Rückgrat ganz oder zumindest zum Teil aus Organosiloxanen besteht, beschrieben u.a. in WO 96/34030.

Bei einem aus dem Stand der Technik bekannten Herstellverfahren für alkoxysilanterminierte Präpolymere wird von Polyolen, z.B. von Polyether- oder Polyesterpolyolen, ausgegangen, die in einem ersten Reaktionsschritt mit einem Überschuss eines Di- oder Polyisocyanates umgesetzt werden. Anschließend werden die dabei erhaltenen isocyanatterminierten Präpolymere mit aminoalkylfunktionelien Alkoxysilanen zu dem gewünschten alkoxysilanterminierten Präpolymer umgesetzt. Alternativ können alkoxysilanterminierte Präpolymere auch hergestellt werden, indem ein Polyol oder ein OHfunktionktionelles Polyurethan, wie es durch die Reaktion von Polyolen mit einem Unterschuss an Di- oder Polyisocyanaten herstellbar ist, mit einem isocyanatoalkylfunktionellen Alkoxysilan umgesetzt wird.

Diese Herstellverfahren sind beispielsweise in EP 1 421 129 oder WO 2005/000931 beschrieben. Auch weitere Herstellverfahren, z.B. die in WO 02/034838 beschriebene Umsetzung von aminofunktionellen Polyolen mit Carbamatoalkyl-alkoxysilanen, sind vorstellbar.

Des Weiteren sind auch alkoxysilanfunktionelle Präpolymere mit einem Poly(meth)acrylatrückgrat bekannt. Diese werden typischerweise durch eine Copolymerisation von (Meth)Acryloxyalkylalkoxysilanen mit anderen (Meth)Acrylmonomeren und/oder weiteren ungesättigten Monomerbausteinen, wie z.B. Styrol, aufgebaut. Zudem können alkoxysilanfunktionelle Polymere auch durch nachträgliches Aufpfropfen von ungesättigten Alkoxysilanen, z.B. von Vinyl- oder (Meth)Acrylsilanen, hergestellt werden.

Nachteilig an den beschriebenen Präpolymeren ist die geringe Funktionalisierungsdichte der nur einfach oder maximal terminal in α,ω-Position mit Silylgruppen terminierten Präpolymere. Die Silylmodifizierung ist oftmals ungenügend, um den angestrebten Effekt einer guten und dauerhaften Substrathaftung wirksam zu adressieren. Daher bezieht sich die Lehre der WO 2008/058955 auf die Option, freie monomere Silane den nur in α,ω-Position mit Silylgruppen terminierten Präpolymeren als zweite Komponente hinzuzufügen, um die eingangs diskutierten gewünschten Effekte (Haftvermittlung, Trocknung, Vernetzung u.ä.) hervorzurufen. Sichergestellt ist hiermit keinesfalls der zielgerichtete Einbau von Silylankergruppen an den Stellen des Polymers, die deren positiver Wirkung bedürfen.

Es bestand mithin die Aufgabe, die Nachteile der Verbindungen des Standes der Technik zu überwinden und verbesserte, in ihrer chemischen Struktur flexibel und optimal auf die jeweiligen Substrate und Verarbeitungsbedingungen zugeschnittene Beschichtungsmittel zur Verfügung zu stellen.

Überraschenderweise wurde nun gefunden, dass man die hier dargelegten technischen Beschränkungen und Nachteile überwinden kann, indem man zur Oberflächenmodifizierung und Beschichtung von Partikeln und anderen flächigen Untergründen Zusammensetzungen enthaltend als Komponente eine oder mehrere gemischte härtbare hydroxygruppenhaltige Silylpolyether mit mindestens einer nicht-terminalen Silylfunktionen, bevorzugt mehr als einer nicht-terminalen und besonders bevorzugt mehr als einer nicht-terminalen sowie gleichzeitig mindestens einer terminalen Silylfunktion im Molekül. Insbesondere enthalten sie mehr als eine (1) Alkoxysilyfunktion pro gegenüber Epoxidgruppen reaktivem Kettenende. Diese Verbindungen sind durch Alkoxylierung epoxidfunktioneller Alkoxysilane an Doppelmetallcyanid (DMC)-Katalysatoren zugänglich sind und in der noch nicht offengelegten Schrift DE 10 2008 000360.3 beschrieben werden, die hiermit vollumfänglich als Teil und Gegenstand dieser Offenbarung eingeführt wird.

Diese neuen Silylgruppen tragende Hydroxylverbindungen gemäß der Formel 1, die sowohl Alkoxysilanfunktionen innerhalb der Sequenz der Oxyalkyleneinheiten der Polyetherkette als auch neue Alkoxysilanfunktionen in ihren Termini aufweisen können, erlauben es, die Ankergruppendichte in dem angestrebten Präpolymer nach Belieben, d.h., angepasst auf die jeweilige anwendungstechnische Fragestellung einzustellen. Jene Polyetherstrukturen können auch über eine SiC- oder SiOC-Bindung an lineare oder verzweigte Polysiloxankörper angebunden sein, wie in DE 10 2008 044373.5 dargestellt.

Diese neuartigen reaktiven Polyether und/oder Polyethersiloxane stellen auf Grund ihrer hydrolyseempfindlichen und zur Vernetzung neigenden Alkoxysilylgruppen härtbare Polymere dar. Deren Vernetzung zu festen duroplastischen Endprodukten oder deren chemische Anbindung auf reaktive Oberflächen, z.B. auf Partikeloberflächen, erfolgt auf einfache Weise wahlweise unter Zusatz von Wasser, Säure oder Base als Beschleuniger, wobei durch Erhöhung der Temperatur während des Härtungsvorgangs die Härtungszeit gesteuert werden kann. Somit kann der Polymeraufbau dieser vernetzbaren Polyether und Polyethersiloxane je nach und Art des Starters und Siloxankörpers sowie nach Art, Menge und Abfolge der einsetzbaren Epoxidmonomere auf mannigfaltige Weise variiert werden, um auf diesem Weg wichtige anwendungstechnische Produkteigenschaften abhängig vom jeweiligen Verwendungszweck maßzuschneidern. So lassen sich beispielsweise durch eine Variation des Anteils an Alkoxysilan-Einheiten in der Polymerkette die Vernetzungsdichte und damit das mechanische und physiko-chemische Eigenschaftsprofil der ausgehärteten Systeme in weiten Grenzen beeinflussen.
Überraschenderweise sind hier selbst mit beachtlicher Alkoxysilyl-Funktionalisierungsdichte ausgestattete Polyether und Polyethersiloxane bei Raumtemperatur und Normaldruck niedrigviskose, gut handhabbare Flüssigkeiten mit Viskositäten von typischerweise unter 1000 mPas, so dass keinerlei Einschränkungen im Hinblick auf die Dosierung dieser Komponente zu verzeichnen sind.
Diese Beobachtung differenziert die erfindungsgemäße Lehre von der in der WO 2008/058955 dargelegten Vorgehensweise, die auf das Einbringen freier Silanmonomerer als Formulierungsbestandteile in den Endrezepturen abstellt, um zu gewährleisten, dass die notwendige Vernetzungsdichte bei gleichzeitig niedriger Verarbeitungsviskosität erzielt wird. Die im Hinblick auf ihre strukturelle Vielfalt kaum einzugrenzenden Alkoxysilylgruppen aufweisenden Polyether und deren Siloxancopolymere eröffnen dem in der Polymerchemie vertrauten Fachmann durch den Einbau z.B. von Ester-, Carbonat- und aromatischen Strukturelementen in die Polyetherstruktur eine Gestaltungsfreiheit, die nahezu beliebige anwendungstechnische Bedürfnisse adressiert. **Noch größer ist die Vielfalt an möglichen** Alkoxysilylpolyether-Siloxan-Copolymerstrukturen, da jeder organische Rest ein- oder mehrfach, terminal oder seitenständig, an ein lineares oder unterschiedlich stark verzweigtes, in seiner Molmasse variables und durch andere Kohlenstoffreste ggf. zusätzlich modifiziertes Polysiloxangerüst gebunden ist, wobei die chemische Verknüpfung des organischen Polyetheranteils wahlweise über eine SiC oder eine SiOC Bindung erfolgt. Ebenso sind beliebige Mischungen von Alkoxysilylpolyethern mit Alkoxysilylgruppen tragenden Siliconpolyethern einsetzbar.

Herkömmliche organische Polymere, wie beispielsweise in der WO 2005/100482 beschrieben, enthalten hingegen innerhalb ihrer Hauptkette oder den Hauptketten keine Silylgruppen, sondern nur an den jeweiligen Kettenenden der Polymerketten. So sind in einem linearen Polymer nur die beiden Enden des Polymers silyl-terminiert, ein solches Polymer wird im Folgenden als divalent oder zweiwertig bezeichnet. Bei der Verwendung eines beispielsweise Glycerin-gestarteten Polymers können sich ausgehend von den drei Hydroxygruppen des Glycerins drei unabhängige silylgruppenfreie Polymerstränge bilden,
wobei wiederum erst am Strangende eine Silylgruppe als Terminus angebracht sein kann. Ein derart verzweigtes Polymer mit drei Enden wird im Folgenden als trivalent oder dreiwertig bezeichnet. Analog werden Polymere mit vier Enden als vierwertig bezeichnet.

Das Fehlen weiterer Silylgruppen in den Strukturen der DE 10 2004 018548 innerhalb der Basispolymerkette, z.B. als Seitengruppen, begrenzt die Vernetzungsdichte sowie die Haftung nach der Aushärtung. Bei einer Vernetzung über Seitengruppen kann die Haftung aufgrund zusätzlicher Ankergruppen auf dem jeweiligen Substrat erhöht werden, **oder aber durch die geeignete Wahl der** Silylfunktionalisierung anwendungsabhängig angepasst werden. Durch unter anderem auch die geeignete Strukturselektion wird das Zeitfenster der Härtung damit in das Ermessen des Anwenders gestellt; gleichzeitig können so auch Partikel enthaltende Zusammensetzungen mit hervorragender Lagerstabilität zielgerichtet hergestellt werden.

Gegenstand der Erfindung ist daher die Verwendung härtbarer Silylpolyether 1 als Bestandteil von Zusammensetzungen, verwendbar als Modifizierungsmittel für Oberflächen, hergestellt durch DMC-katalysierte Alkoxylierung von epoxyfunktionellen Alkoxysilanen.
Die Oberflächen können mikroskopisch, beispielsweise in Form von Partikeln oder Partikelagglomeraten, und/oder makroskopisch in Form von Flächengebilden oder Fasern oder ähnlichen dreidimensionalen Körpern ausgebildet sein.

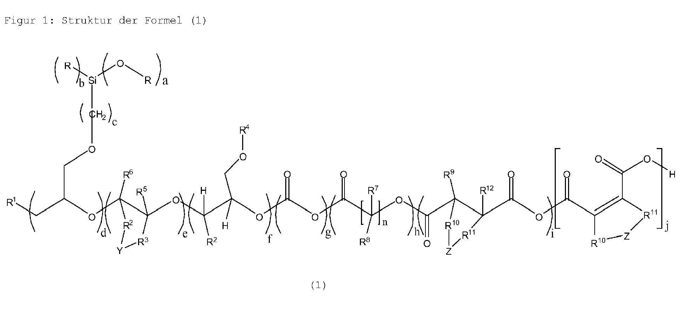

Bevorzugt werden zur Partikelmodifizierung Silylpolyether 1 der Formel (1) - siehe auch Figur 1 - eingesetzt. Diese bestehen aus Alkoxysilylgruppen substituierten Ketten, die durch die Wahl der Fragmente d bis j, entsprechend der durch die Reaktion unter Ringöffnung der Reaktionskomponenten in die Polymerkette eingefügten Fragmente, gezielt hochfunktionalisiert sind und damit für verschiedenartige Anwendungsgebiete zugeschnitten werden können. 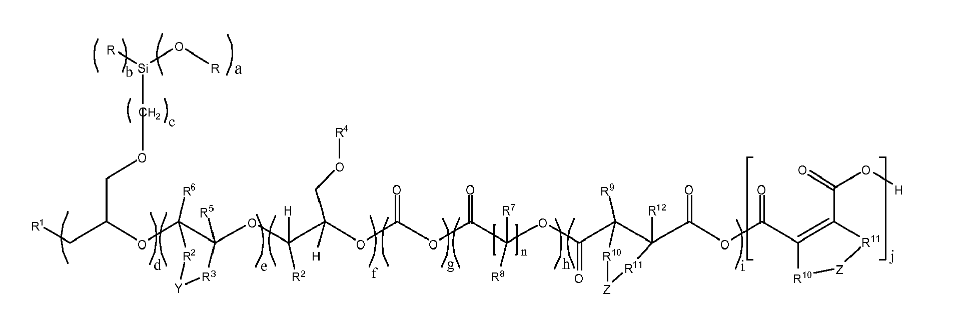 Silylpolyether 1 der Formel (1) (siehe auch Fig. 1)
wobei
- a: eine ganze Zahl von 1 bis 3, vorzugsweise 3 ist,
- b: eine ganze Zahl von 0 bis 2, vorzugsweise 0 bis 1, besonders bevorzugt 0 ist,
die Summe von a und b gleich 3 ist,
- c: eine ganze Zahl von 0 bis 22, bevorzugt von 0 bis 6, besonders bevorzugt gleich 1 oder 3 ist,
- d: eine ganze Zahl von größer 1 bis 1.000, bevorzugt größer 1 bis 100, besonders bevorzugt größer 1 bis 20 und insbesondere größer 1 bis 10 ist, oder größer 10 bis 100 ist,
- e: eine ganze Zahl von 0 bis 10.000, bevorzugt 0 bis 1000, besonders bevorzugt 0 bis 300 und insbesondere 0 bis 100 ist,
- f: eine ganze Zahl von 0 bis 1.000, bevorzugt 0 bis 100, besonders bevorzugt 0 bis 50 und insbesondere 0 bis 30 ist,
- g: eine ganze Zahl von 0 bis 1.000, bevorzugt 0 bis 200, besonders bevorzugt 0 bis 100 und insbesondere 0 bis 70 ist,
- h, i und j: ganze Zahlen von 0 bis 500, bevorzugt 0 bis 300, besonders bevorzugt 0 bis 200 und insbesondere 0 bis 100 ist,
und mit der Maßgabe, dass die Fragmente mit den Indices d bis j untereinander frei permutierbar, d.h.
in der Sequenz innerhalb der Polyetherkette gegeneinander austauschbar sind,
- n: eine ganze Zahl zwischen 2 und 8 ist und
- R: einen oder mehrere gleiche oder verschiedene Reste, ausgewählt aus linearen oder verzweigten, gesättigten, einfach oder mehrfach ungesättigten Alkylresten mit 1 bis 20, insbesondere 1 bis 6 Kohlenstoffatomen oder Halogenalkylgruppen mit 1 bis 20 Kohlenstoffatomen darstellt. Bevorzugt entspricht R Methyl-, Ethyl-, Propyl-, Isopropyl-, n-Butyl- und sek.-Butylgruppen, und insbesondere Ethyl- oder Methylgruppen,
mit
- R¹: gleich einem gesättigten oder ungesättigten, gegebenenfalls verzweigten Rest, der vorzugsweise über ein Sauerstoffatom angebunden ist, oder stellt einen Polyetherrest vom Typ einer Alkoxy-, Arylalkoxy- oder Alkylarylalkoxygruppe dar, bei der die Kohlenstoffkette durch Sauerstoffatome unterbrochen sein kann, oder R¹ eine ggf. einfach oder mehrfach annelierte aromatische Aryloxy-Gruppe, oder eine siliciumhaltige Verbindung, insbesondere ein Siloxanrest ist, der alkyl- und/oder arylgruppen- und/oder polyethersubstituiert sein kann,
- R²: oder R³, sowie R⁵ oder R⁶ gleich oder auch unabhängig voneinander H oder ein gesättigter oder gegebenenfalls einfach oder mehrfach ungesättigter, auch weiter substituierter, gegebenenfalls ein- oder mehrwertiger Kohlenwasserstoffrest, wobei für die Reste R⁵ oder R⁶ gilt, dass sie gleich einem einwertigen Kohlenwasserstoffrest sind. Der Kohlenwasserstoffrest kann cycloaliphatisch über das Fragment Y verbrückt sein; Y kann nicht vorhanden sein, oder aber eine Methylenbrücke mit 1 oder 2 Methyleneinheiten sein, ist Y nicht vorhanden, so sind R² oder R³ unabhängig voneinander gleich ein linearer oder verzweigter Rest mit 1 bis 20, bevorzugt 1 bis 10 Kohlenstoffatomen, besonders bevorzugt ein Methyl-, Ethyl-, Propyl- oder Butyl-, Vinyl-, Allylrest oder Phenylrest. Vorzugsweise ist zumindest einer der beiden Reste R² oder R³ Wasserstoff. R²-R³ kann eine -CH₂CH₂CH₂CH₂-Gruppe, Y damit eine -(CH₂CH₂-)-Gruppe sein. Die Kohlenwasserstoffreste R² und R³ können ihrerseits weiter substituiert sein und funktionelle Gruppen wie Halogene, Hydroxylgruppen oder Glycidyloxypropylgruppen tragen.
- R⁴: entspricht einem linearen oder verzweigten Alkylrest von 1 bis 24 Kohlenstoffatomen oder einem aromatischen oder cycloaliphatischen Rest, der gegebenenfalls seinerseits Alkylgruppen tragen kann.
- R⁷ und R⁸: sind unabhängig voneinander entweder Wasserstoff, Alkyl-, Alkoxy-, Aryl- oder Aralkylgruppen, die unter Ringöffnungspolymerisation zu vernetzbaren, Alkoxysilangruppen enthaltenden Polyetherestern copolymerisiert werden.
- R⁹, R¹⁰, R¹¹ und R¹²: sind unabhängig voneinander entweder Wasserstoff, Alkyl-, Alkenyl-, Alkoxy-, Aryl- oder Aralkylgruppen. Der Kohlenwasserstoffrest kann cycloaliphatisch oder aromatisch über das Fragment Z verbrückt sein, wobei Z sowohl einen divalenten Alkylen- als auch Alkenylenrest darstellen kann.

Es kann vorteilhaft sein, wenn die Silylpolyether der Formel (1) solches sind, die ausschließlich Reste R¹ aufweisen, die Siliziumatome enthalten, oder solche, die ausschließlich Reste R¹ aufweisen, die keine Siliziumatome aufweisen.

Die verschiedenen Monomereinheiten sowohl der Fragmente mit den Indexzahlen d bis j als auch der eventuell vorhandenen Polyoxyalkylenkette des Substituenten R¹ können untereinander blockweise aufgebaut sein oder aber auch einer statistischen Verteilung unterliegen. Die in den hier angeführten Formeln wiedergegebenen Indexzahlen und die Wertbereiche der angegebenen Indizes verstehen sich daher als die Mittelwerte der möglichen statistischen Verteilung der tatsächlichen vorhandenen Strukturen und/oder deren Mischungen. Dies gilt auch für als solche an sich exakt wiedergegebene Strukturformeln, wie beispielsweise für Formel (1).

Ganz besonders bevorzugt sind 3-Glycidyloxyalkyltrialkoxysilane und 3-Glycidyloxyalkyldialkooxyalkylsilane als Monomere.

Wie ²⁹Si-NMR- und GPC-Untersuchungen ergeben, bedingt das verfahrensbedingte Vorhandensein von kettenendständigen OH-Gruppen die Möglichkeit zu Umesterungsreaktionen am Siliziumatom sowohl während der DMC-katalysierten Herstellung als auch z.B. in einem nachgeschalteten Prozessschritt. Dabei wird formal der über ein Sauerstoffatom an das Silizium gebundene Alkylrest R gegen einen langkettigen modifizierten Alkoxysilylpolymerrest ausgetauscht. Bimodale wie auch multimodale GPC Kurven belegen, dass die Alkoxylierungsprodukte neben den nicht umgeesterten Spezies, wie sie in Formel (1) wiedergegeben sind, solche mit der doppelten, zum Teil dreifachen oder gar vielfachen Molmasse enthalten. Formel (1) gibt mithin die komplexe chemische Realität nur vereinfacht wieder.

Somit enthalten die Zusammensetzungen auch Verbindungen, in denen die Summe der Indices (a) plus (b) in Formel (1) im statistischen Mittel kleiner als 3 ist, da ein Teil der OR-Gruppen durch Silylpolyethergruppen ersetzt werden kann. Die Zusammensetzungen enthalten somit Spezies, die am Siliziumatom unter Abspaltung von R-OH und Kondensationsreaktion mit der reaktiven OH-Gruppe eines weiteren Moleküls der Formel (1) ausgebildet werden. Diese Reaktion kann mehrfach ablaufen bis z.B. alle RO-Gruppen am Silizium gegen weitere Moleküle der Formel (1) ausgetauscht sind. Das Vorhandensein von mehr als einem Signal in typischen ²⁹Si-NMR-Spektren dieser Verbindungen untermauert das Auftreten von Silylgruppen mit unterschiedlichem Substitutionsmuster. Die angegebenen Werte und Vorzugsbereiche für die Indizes a bis j sind somit auch nur als Mittelwerte über die verschiedenen, einzeln nicht fassbaren Spezies zu verstehen.

Je nach verwendetem epoxidfunktionellem Alkoxysilan und evtl. eingesetzten weiteren Monomeren, sowie evtl. auch Kohlendioxid können unterschiedlich aufgebaute Silylpolyether 1 hergestellt werden, sowie deren beliebig aufgebaute Gemische. Bevorzugt ist die Alkoxysilaneinheit **in der Verbindung der Formel** (1) **eine** Trialkoxysilyleinheit.

Die Silylpolyether 1 der Formel (1) können allein oder in beliebiger Kombination, abgemischt mit monomeren Alkoxysilanen, alkoxysilylterminierten Präpolymeren, Härtungskatalysatoren sowie weiteren Zusatz- und Hilfsstoffen in der Mischung mit den zu beschichtenden festen Partikeln bzw. makroskopischen Oberflächen verwendet werden.

Ein weiterer Gegenstand der vorliegenden Erfindung sind deshalb auch Zusammensetzungen, die neben den Silylpolyethern 1 (1) auch andere Silylverbindungen, insbesondere solche der Formel (2), enthalten. Die bevorzugt eingesetzten Silylpolyether 1 der Formel (1) sind solche mit d > 1. Sie ermöglichen dank ihrer zahlreichen reaktiven Ankergruppen eine verbesserte Haftung auf der jeweiligen makroskopischen oder auch Partikeloberfläche, als dies beim Stand der Technik unter Einsatz üblicher monomerer Silane wie denen gemäß Formel (2) **oder der Verwendung von** α,ω-silylterminierten Präpolymeren möglich ist.

Durch Anpassung des strukturellen Aufbaus des Polymergerüsts, also zum Beispiel durch Einbringung von Ester- oder Carbonatgruppen oder aromatischen Strukturelementen, durch Einstellung einer gewünschten Molmasse, wahlweise block- oder statistischer Monomerabfolge bis hin zur Alkoxysilylfunktionalität kann das Eigenschaftsprofil flexibel an die jeweiligen Anforderungen des Gesamtsystems angepasst werden, um z.B. eine ideale Haftung auf der Partikeloberfläche zu gewährleisten, die Verarbeitungseigenschaften (z.B. Viskosität) während der Partikelmodifizierung einzustellen oder für eine Kompatibilisierung der Partikel in der jeweiligen Matrix zu fördern.

Bevorzugt ist die Verwendung härtbarer Silylpolyether 1 mit mehr als 1 Alkoxysilylfunktion, ganz besonders bevorzugt solche mit im Mittelwert mehr als einer derartigen Silylgruppe pro terminaler Hydroxylgruppe in Zusammensetzungen verwendbar zur Oberflächenmodifikation.

Die Silylpolyether 1 gewähren die synthetische Freiheit, zwischen Alkoxysilylgruppen aufweisenden Polyoxyalkylenverbindungen zu wählen, die die hydrolysierend vernetzbaren Alkoxysilylfunktionen sowohl terminal, als auch isoliert, blockartig kumuliert als aber auch statistisch eingestreut in die Polyoxyalkylenkette enthalten. Derartige Silylpolyether 1 der Formel (1), zeichnen sich dadurch aus, dass sie hinsichtlich Strukturaufbau und Molmasse gezielt und reproduzierbar hergestellt werden können. Die Sequenz der Monomereinheiten kann in weiten Grenzen variabel gestaltet werden. Epoxidmonomere können beliebig blockartig aneinander gereiht oder statistisch in die Polymerkette eingebaut sein. Die durch die Reaktion unter Ringöffnung der Reaktionskomponenten in die entstehende Polymerkette eingefügten Fragmente sind in ihrer Sequenz untereinander frei permutierbar, mit der Einschränkung, dass cyclische Anhydride sowie Kohlendioxid statistisch insertiert, also nicht in homologen Blöcken, in der Polyetherstruktur vorliegen.

Wie dem Fachmann bekannt, geschieht die Vernetzung oder Härtung von Alkoxysilylgruppen in einem zweistufigen chemischen Prozess, bei dem in einem ersten Schritt in Gegenwart von Wasser, wobei auch Luftfeuchtigkeit ausreichen kann, die am Silizium gebundenen Alkoxygruppen als korrespondierende Alkohole abgespalten und SiOH-Gruppen ausgebildet werden. Letztere kondensieren im Falle der Selbstkondensation anschließend unter Ausbildung von Si-O-Si-Brücken miteinander und bilden polymere Werkstoffe. Bevorzugt reagieren die SiOHfunktionellen Intermediate mit reaktive Gruppen aufweisenden Substraten, z.B. besonders gut mit OH-Funktionen tragenden silikatischen Oberflächen, und führen zu einer exzellenten chemischen Verankerung auf dem jeweiligen Untergrund. Die Härtungsgeschwindigkeit lässt sich auf vielfältige Weise durch Zusatz von Katalysatoren oder Temperaturvariation beeinflussen.

Werden als Silylpolyether 1 solche eingesetzt, die am Siliziumatom mehr als 1 der hochfunktionalisierten Polyalkylenetherfragmenten gebunden enthalten, so liegen hochfunktionalisierte Verbindungen vor, bei denen Polyetherketten, die jeweils von einem Startalkohol der Formel (3) R¹-H (das H gehört zur OH-Gruppe der als Starter eingesetzte OH-gruppenhaltigen Verbindung, hier als Startalkohol bezeichnet) abgeleitet sind und die in ihrer Abfolge die frei permutierbaren Fragmente enthalten, die durch die Reaktion unter Ringöffnung der Reaktionskomponenten in die entstehende Polymerkette eingefügt wurden, über -CH₂-O- (CH₂)_{c}-Si- (CH₂)_{c}-O-CH₂-Brücken miteinander verknüpft sind. Es handelt sich um hochkomplexe, hochfunktionalisierte Strukturen. Auch hier lassen sich die Funktionalitäten gezielt auf ein gewünschtes Anwendungsgebiet einstellen. Der Verzweigungsgrad und die Komplexität der erhaltenen Polymerstrukturen steigen mit zunehmender Epoxyfunktionalität der Silylmonomere. Die Kettenlänge der als Startverbindung einsetzbaren Alkoxy-, Arylalkoxy- oder Alkylarylalkoxygruppen aufweisenden Polyetherreste ist beliebig. Vorzugsweise enthält die Polyether-, Alkoxy-, Arylalkoxy- oder Alkyarylalkoxygruppe 1 bis 1.500 Kohlenstoffatome, besonders bevorzugt 2 bis 300 Kohlenstoffatome, insbesondere 2 bis 100 Kohlenstoffatome. Als OH-funktionelle Startverbindungen R¹-H (3) werden vorzugsweise Verbindungen mit Molmassen von 18 bis 10.000 g/mol, insbesondere 50 bis 2000 g/mol und mit 1 bis 8, bevorzugt mit 1 bis 4 Hydroxylgruppen eingesetzt. Soll allerdings eine Siloxangruppierung als R¹ in den Silylpolyether eingeführt werden, so werden beispielsweise α, ω-Dihydroxypolysiloxane, Wasserstoffsiloxane oder hydroxylfunktionelle Polyethersiloxane als Ausgangsverbindungen verwendet. Die Siloxankörper dieser Reste R¹ entsprechen den in Formel (5) bzw. (6) beschriebenen Strukturen.

Ganz besonders geeignete Modifizierungsmittel für Oberflächen und/oder Beschichtungen sind die in der noch nicht vorveröffentlichten Schrift DE 10 2008 044373.5 beschriebenen Alkoxysilaneinheiten tragenden Polyethersiloxane. Der Inhalt der Beschreibung und der Ansprüche ist hiermit vollumfänglich als Bestandteil dieser Offenbarung anzusehen.

Wird als R¹ damit ein (Poly-)Siloxanrest in das Molekül eingeführt, so werden alkoxysilylfunktionelle Polyethersiloxane erfindungsgemäß verwendet. Diese alkoxysilylfunktionellen Polyethersiloxane und deren Mischungen lassen sich nach zwei verschiedenen Verfahren herstellen, wie in DE 10 2008 0044373.5 dargestellt:
1) Alkoxylierung von Siliconpolyethercopolymeren bzw. Polysiloxanen mit epoxyfunktionellen Alkoxysilanen an Doppelmetallcyanid-Katalysatoren und/oder
2) Hydrosilylierende Verknüpfung von ungesättigten Alkoxysilylgruppen tragenden Polyethern, die zuvor durch eine Alkoxylierung der entsprechenden ungesättigten Startverbindungen mit epoxyfunktionellen Alkoxysilanen an DMC-Katalysatoren gewonnen wurden.

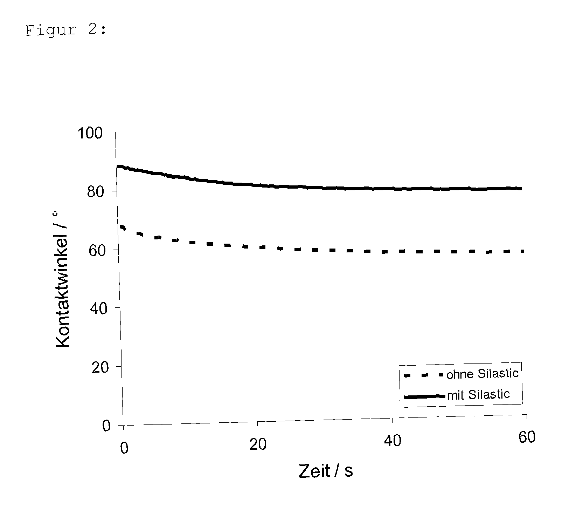

Die alkoxysilylfunktionellen Polyethersiloxane sind Verbindungen gemäß Formel (5) und deren Mischungen, 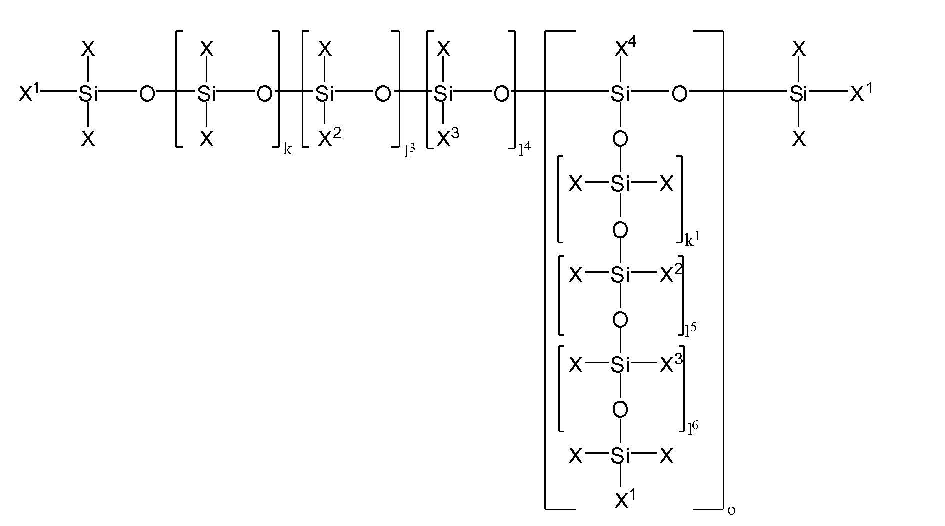 wobei
- X: ein linearer, cyclischer oder verzweigter, aliphatischer oder aromatischer, gesättigter oder ungesättigter Kohlenwasserstoffrest mit 1 bis 20 C-Atomen ist, der ggfs. Heteroatome wie Sauerstoff, Stickstoff, Phosphor oder Schwefel enthalten kann, der jedoch vorzugsweise eine Methylgruppe ist,
- X¹: wahlweise X, X² oder X³ ist,
- X²: ein Alkoxysilylgruppen tragender OH-funktioneller, ggfs. Ester- oder Carbonat-modifizierter Polyoxyalkylenrest der Formel (5a) - siehe auch Figur 2 - ist, 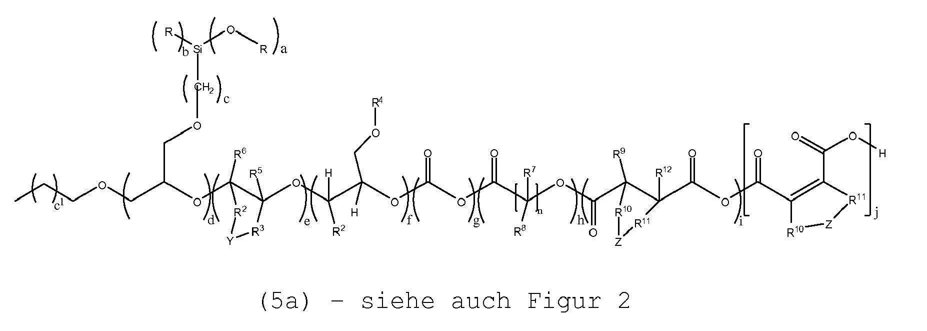
- X³: ein endständig veretherter Polyoxyalkylenrest der Formel (5b) ist, 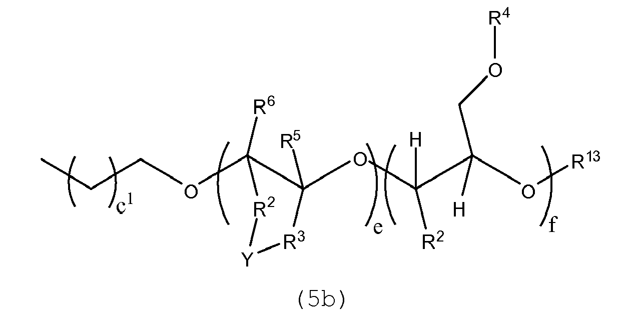 wobei
- R¹³: wahlweise eine Alkylgruppe mit 1 bis 18 C-Atomen, vorzugsweise Methyl ist,
oder ein mit einer monofunktionellen Carbonsäure endständig veresterter Polyoxyalkylenrest der Formel (5c) ist, 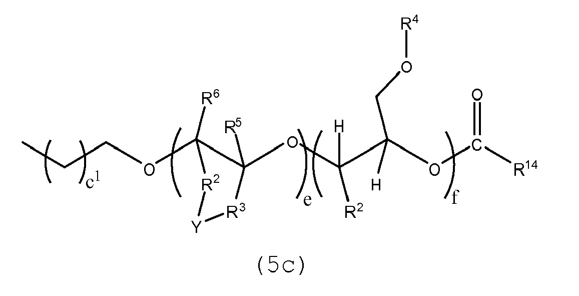 wobei
- R¹⁴: ein gesättigter oder ein ein- oder mehrfach ungesättigter, entweder linearer oder verzweigter, aliphatischer oder aromatischer Kohlenwasserstoffrest mit 1-30 Kohlenstoffatomen ist, der seinerseits OH-Gruppen tragen kann, vorzugsweise ein Methylrest ist,
- X⁴: entweder X¹ oder dem Fragment der Formel (5d) entspricht 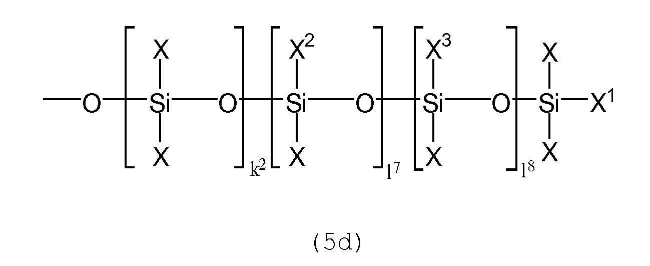 wobei
k, k¹ und k² unabhängig voneinander ganze Zahlen von 0 bis 500, vorzugsweise von 10 bis 200, insbesondere 15 bis 100 sind,
l³, l⁴, l⁵, l⁶, l⁷ und l⁸ unabhängig voneinander ganze Zahlen von 0 bis 60, vorzugsweise von 0 bis 30, insbesondere von 0 bis 25 sind,
o eine ganze Zahl von 0 bis 10, vorzugsweise von 0 bis 3 ist,
mit der Maßgabe, dass
X¹ mindestens einmal gleich X² ist, falls die Summe aus l³, l⁵ und l⁷ Null ist,
und dass die Summe aus l³, l⁵ und l⁷ mindestens 1 ist, wenn X¹ ungleich X² ist,
wobei
a eine ganze Zahl von 1 bis 3, vorzugsweise 3 ist,
b eine ganze Zahl von 0 bis 2, vorzugsweise 0 bis 1, besonders bevorzugt 0 ist, die Summe von a und b gleich 3 ist,
c eine ganze Zahl von 0 bis 22, bevorzugt von 0 bis 6, besonders bevorzugt gleich 1 oder 3 ist,
c¹ eine ganze Zahl von 0 bis 24, vorzugsweise von 0 bis 12, besonders bevorzugt von 0 bis 8, ganz besonders bevorzugt von 0 bis 4, insbesondere gleich 1 ist,
d eine ganze Zahl von größer 1 bis 1.000, bevorzugt größer 1 bis 100, besonders bevorzugt größer 1 bis 20 und insbesondere größer 1 bis 10 ist, oder größer 10 bis 100 ist,
e eine ganze Zahl von 0 bis 10.000, bevorzugt 0 bis 1000, besonders bevorzugt 0 bis 300 und insbesondere 0 bis 100 ist,
n eine ganze Zahl von 2 bis 8 ist und
f, g, h, i und j jeweils ganze Zahlen von 0 bis 500, bevorzugt 0 bis 300, besonders bevorzugt 0 bis 200, insbesondere 0 bis 100 sind,
mit der Maßgabe, dass die Fragmente mit den Indices d bis j untereinander frei permutierbar, d.h. in der Sequenz innerhalb der Polyetherkette gegeneinander austauschbar sind und wobei die verschiedenen Monomereinheiten der Fragmente mit den Indexzahlen d bis j untereinander blockweise aufgebaut sein oder aber auch einer statistischen Verteilung unterliegen können und mit der Maßgabe, dass die Fragmente mit den Indices k, k¹, k², l³, l⁴, l⁵, l⁶, l⁷, l⁸ und o untereinander frei permutierbar, d.h. innerhalb der Siloxankette gegeneinander austauschbar sind und wahlweise statistisch verteilt oder blockartig aneinandergereiht vorliegen können.
R stellt einen oder mehrere gleiche oder verschiedene Reste, ausgewählt aus linearen oder verzweigten, gesättigten, einfach oder mehrfach ungesättigten Alkylresten mit 1 bis 20, insbesondere 1 bis 6 Kohlenstoffatomen oder Halogenalkylgruppen mit 1 bis 20 Kohlenstoffatomen dar, bevorzugt eine Methyl-, Ethyl-, Propyl-, Isopropyl-, n-Butyl- oder sek.-Butylgruppe.
R² oder R³, sowie R⁵ oder R⁶ sind gleich oder unabhängig voneinander H oder ein gesättigter oder gegebenenfalls einfach oder mehrfach ungesättigter, auch weiter substituierter, gegebenenfalls ein- oder mehrwertiger Kohlenwasserstoffrest, wobei für die Reste R⁵ oder R⁶ gilt, dass sie gleich einem einwertigen Kohlenwasserstoffrest sind. Der Kohlenwasserstoffrest kann cycloaliphatisch über das Fragment Y verbrückt sein; Y kann nicht vorhanden sein, oder aber eine Methylenbrücke mit 1 oder 2 Methyleneinheiten sein; ist Y gleich 0, so sind R² oder R³ unabhängig voneinander gleich ein linearer oder verzweigter Rest mit 1 bis 20, bevorzugt 1 bis 10 Kohlenstoffatomen, besonders bevorzugt ein Methyl-, Ethyl-, Propyl- oder Butyl-, Vinyl-, Allylrest oder Phenylrest. Vorzugsweise ist zumindest einer der beiden Reste in R² oder R³ Wasserstoff. Die Kohlenwasserstoffreste R² und R³ können ihrerseits weiter substituiert sein und funktionelle Gruppen wie Halogene, Hydroxylgruppen oder Glycidyloxypropylgruppen tragen.
R⁴ ist ein linearer oder verzweigter Alkylrest von 1 bis 18 Kohlenstoffatomen, der an einen aromatischen oder cycloaliphatischen Rest gebunden sein kann.
R⁷ und R⁸ sind unabhängig voneinander entweder Wasserstoff, Alkyl-, Alkoxy-, Aryl- oder Aralkylgruppen.
R⁹, R¹⁰, R¹¹ und R¹² sind unabhängig voneinander entweder Wasserstoff, Alkyl-, Alkenyl-, Alkoxy-, Aryl- oder Aralkylgruppen, wobei der Kohlenwasserstoffrest cycloaliphatisch oder aromatisch über das Fragment Z verbrückt sein, wobei Z sowohl einen divalenten Alkylen- als auch Alkenylenrest darstellen kann.

Die durch Formel (5) beschriebenen Polyethersiloxane schließen die gegebenenfalls prozessbedingt enthaltenen Nebenprodukte wie freie Überschusspolyether oder Umlagerungsprodukte mit ein.

Die verschiedenen Monomereinheiten innerhalb der Siloxankette bzw. innerhalb der damit verknüpften Polyetherkette können untereinander wahlweise blockweise oder statistisch aufgebaut sein. Die in den hier angeführten Formeln wiedergegebenen Indexzahlen und die Wertebereiche der angegebenen Indizes verstehen sich als die Mittelwerte der möglichen statistischen Verteilung der tatsächlichen isolierten Strukturen und/oder deren Mischungen. Dies gilt auch für als solche an sich exakt wiedergegebenen Strukturformeln.

Ganz besonders bevorzugt sind 3-Glycidyloxyalkyltrialkoxysilane und 3-Glycidyloxyalkyldialkooxyalkylsilane als Monomere.

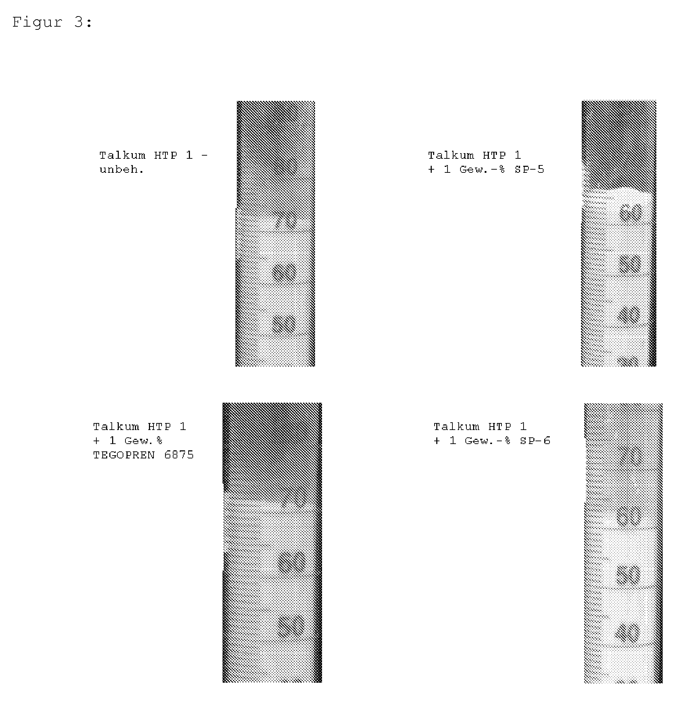

Die Polyethersiloxane mit Alkoxysilylfunktionalisierung der Formel (5) stellen zumeist kammartig verzweigte Copolymere dar, in denen die Polyetherketten jeweils über SiC-Bindungen an das Polysiloxangrundgerüst gebunden sind.
Ebenfalls erfindungsgemäß verwendbar sind lineare Polyether-Siloxan-Polyether-Triblockcopolymere der Formel (6), bei denen die mit Alkoxysilylgruppen ausgestatteten Polyetherketten über eine Si-O-C-Verknüpfung, beispielsweise erhalten aus dehydrogenativen Kopplungsreaktionen, an den Siloxankörper gebunden sind, 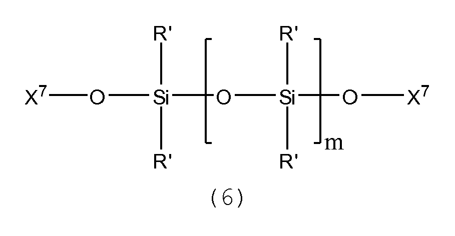 wobei
- R': einem oder mehreren gleichen oder verschiedenen linearen oder verzweigten, gesättigten, einfach oder mehrfach ungesättigten Alkylresten mit 1 bis 20, insbesondere 1 bis 10 Kohlenstoffatomen entspricht,
und
- m: eine ganze Zahl von 0 bis 5000, bevorzugt 2 bis 5000, besonders bevorzugt von 5 bis 4000 ist und insbesondere 9 bis 3000 ist, und
- X⁷: dem Polyetherfragment der Formel (6a) - siehe auch Figur 3 - entspricht.

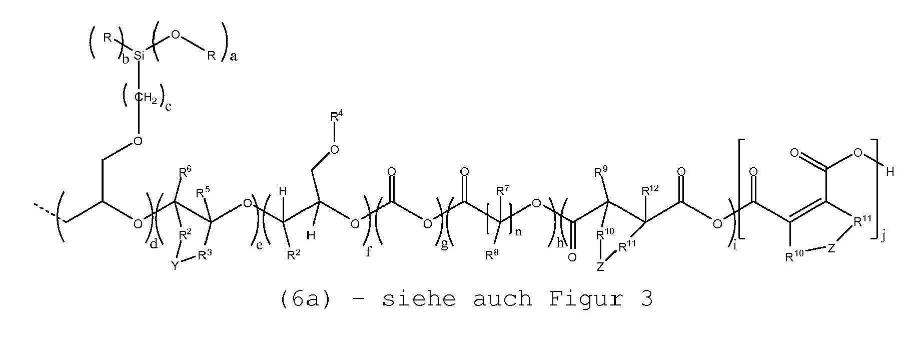

Die Substituenten R, R²-R¹², die Reste Y und Z sowie die Indizes a, b, c, d, e, f, g, h, i, j und n entsprechen den zuvor für die Verbindungen der Formel (5a) genannten Definitionen.

Die in den Formeln (5) bis (5d) und (6) sowie (6a) wiedergegebenen Indexzahlen und die Wertbereiche der angegebenen Indizes verstehen sich als die Mittelwerte der möglichen statistischen Verteilung der tatsächlichen vorhandenen Strukturen und/oder deren Mischungen.

Die Alkoxysilanpolymere der Formeln (1), beziehungsweise die Siloxangruppen enthaltenden Strukturen der Formeln (5) und (6) können allein oder in beliebiger Kombination, abgemischt mit monomeren Alkoxysilanen, alkoxysilylterminierten Präpolymeren, alkoxysilylmodifizierten Siloxanen, wie sie z.B. durch Hydrosilylierung von Wasserstoffsiloxanen mit vinylsubstituierten Alkoxysilanen gewonnen werden können, Siliconharzen, Härtungskatalysatoren sowie weiteren Zusatz- und Hilfsstoffen, die sich in der Summe auf 100 Teile ergänzen, verwendet werden.

Die vorgenannten erfindungsgemäß verwendbaren Silanpolymere gemäß Formel (1) können auch als Komponenten in Mischungen mit herkömmlichen monomeren Silanen der Formel (2) eingesetzt werden,

AₓSiB₍₄₋ₓ₎ (2)

wobei A gleiche oder verschiedene nicht hydrolysierbare Gruppen, B = gleiche oder verschiedene hydrolysierbare Gruppen oder Hydroxygruppen und x = 1, 2, 3 oder 4 darstellt. Ebenso können die Silylpolyether 1 dank ihrer niedrigen Viskosität in Kombination mit anderen, beispielsweise höherviskosen konventionellen silanterminierten Polymeren, deren Viskosität meist größer als 10.000 mPas ist, eingesetzt werden, um die Viskosität des Gesamtsystems zu senken und die Verarbeitbarkeit zu verbessern. Hoch alkoxysilylfunktionelle Präpolymere der Formel (1) steigern die Netzwerkdichte, gewährleisten die geforderte gute chemische Anbindung auf den Substraten und führen letztlich zu hochfesten Beschichtungen oder Oberflächenmodifizierungen.

Neben den erfindungsgemäß verwendbaren Alkoxysilylgruppen aufweisenden Verbindungen (1), können den Modifizierungsmitteln auch weitere zur hydrolytischen Reaktivvernetzung befähigte organomodifizierte Additive zugesetzt werden, insbesondere solche, die Organoalkoxysiloxaneinheiten tragen und nicht zwingend durch obige Formeln beschrieben werden.

Dabei können die Fragmente, die durch die Reaktion unter Ringöffnung in die entstehende Polymerkette eingefügt wurden, im Rahmen der vorhergehenden Definitionen blockartig oder statistisch verteilt, nicht nur in der Kette einer Polyether-Struktureinheit vorkommen, sondern auch statistisch verteilt über die Vielzahl der gebildeten und über -CH₂-O-(CH₂)_{c}-Si-(CH₂)_{c}-O-CH₂-Brücken miteinander verbundenen Polyetherstruktureinheiten, vorkommen. Die Mannigfaltigkeit der Strukturvariationen der Verfahrensprodukte gestattet damit keine eindeutige formelmäßige Beschreibung.

Partikeloberflächen solider oder auch poröser Partikel können erfindungsgemäß mit aus dem Stand der Technik bekannten Methoden oberflächenbeschichtet werden. Dazu gehört das Aufdüsen der Silylpolyether 1 auf die Partikel unter Vermischung, ggf. unter Vermischung, Verknetung und/oder Erwärmen gegebenenfalls in Gegenwart geeigneter Vernetzungskatalysatoren. Ebenso können die erfindungsgemäßen Silylpolyether rein oder aus geeigneten organischen und/oder anorganischen Lösemitteln auf die Partikeloberflächen aufgebracht werden, wo sie dann unter kovalenter Anbindung ausreagieren können. Ebenso ist es möglich, Emulsionen aus den erfindungsgemäßen Silylpolyethern 1 in geeigneten Medien, ggf. unter Zusatz von Hilfsstoffen, weiterer Modifizierungsmittel und Emulgatoren und/oder Netzmitteln, auf die Partikeloberflächen aufzubringen. Auch die Modifizierung von Partikeloberflächen in einer Matrix (vor-)dispergierter Partikel, beispielsweise von in einem Polymer oder einem Lack (vor-)dispergierten partikulären Füllstoffs oder funktionellen Partikeln, ist durch Zugabe der Silylpolyether zu den entsprechenden Systemen unter Durchmischung möglich, ggf. unter Erwärmung und/oder der Zugabe eines geeigneten Katalysators. In jedem Fall können den Silylpolyethern 1 noch weitere Komponenten, wie beispielsweise monomere, oligomere oder polymere Silane oder reaktive Silylgruppen tragende andere Komponenten sowie nach einem anderen Mechanismus aushärtende oder aufziehende Materialien, wie beispielsweise Acrylate, Epoxide, Isocyanate, Carboxylate, Hydroxide, Lactone, Lactame u. a. m, beigemischt sein. Auch können mehrere der Silylpolyether 1 miteinander gemischt verwendet werden.
Zu den zu modifizierenden Partikeln unterschiedlicher Herkunft, unterschiedlicher Größe bzw. Partikelgrößenverteilung sowie unterschiedlicher Morphologie (sphärisch, plättchenförmig (mit unterschiedlichen Aspektverhältnissen), faserig, fraktal aggregiert, würfel- oder quaderförmig u.a.m) und unterschiedlichen Agglomerationszuständen gehören beispielsweise oxidische Partikel, wie pyrogene Kieselsäure, beispielsweise AEROSIL^{®}e der EVONIK Degussa GmbH, Fällungskieselsäuren, beispielsweise SIPERNAT^{®}e der EVONIK Degussa GmbH, Quarzpartikel und weitere anorganische Oxidpartikel, wie Glaspartikel, Titandioxid, wie z. B. AEROXIDE^{®} TiO₂ P25 und AEROXIDE^{®} TiO₂ P90 der EVONIK Degussa GmbH, Aluminiumoxid, wie z. B. AEROXIDE^{®} Alu C der EVONIK Degussa GmbH, Zirkondioxid und/oder Cerdioxid, Eisenoxide, Kupferoxide u.a.m., silikatische Partikel wie beispielsweise Partikel aus Kaolin, Wollastonit, Talkum, Glimmer, Feldspäten u.a.m., Hydroxide wie Aluminiumtri- und/oder Magnesiumdihydroxid, Böhmit, Hydrotalcit und hydroxydische Eisenpigmente, wie beispielsweise FeO(OH), Carbonate, wie beispielsweise Calciumcarbonat und/oder Dolomit, Metalle wie Eisen, Kupfer, Zink, Nickel, Aluminium, Magnesium u.a.m, Metalllegierungen und/oder kohlenstoffhaltige Materialien, wie beispielsweise Graphit und/oder Ruß u. a. m.
Als organische partikuläre Substrate einsetzbar sind Partikel aus z. B. aus Siliconharzen, organomodifizierten Siliconen, organischen Polymeren und/oder Biopolymeren, organischen Polyelektrolyten, Melamincyanurat u.a.m.
Die unterschiedlichen Partikel können auch im Gemisch oberflächenmodifiziert werden.
Das Verhältnis von Partikelmasse zu Oberflächenmodifikator ist abhängig von der zugänglichen Partikeloberfläche, dem gewünschten Modifizierungsgrad und dem Molekulargewicht des Modifizierungsagenzes. Bezogen auf die Masse der zu modifizierenden Partikel kann das erfindungsgemäße Modifizierungsagenz im Massen-Verhältnis von Partikelmasse:Modifizierungsmittelmasse im Bereich von 1:10 bis 1.000.000:1, bevorzugt von 1:1 bis 10.000:1 und besonders bevorzugt im Bereich von 2:1 bis 1.000:1 liegen.

Betrachtet man das Partikelgewicht im Verhältnis zur gesamten Mischung, die zur Oberflächenmodifizierung eingesetzt wird, bestehend aus Zusammensetzungen enthaltend den oder die Silylpolyether 1, ggf. Katalysator, Lösemittel, weitere Silanverbindungen, sowie sonstiger Hilfsstoffe, so kann das Massen-Verhältnis von Partikelgewicht:Modifizierungsmischung im Bereich von 1:1.000 bis 100.000:1, bevorzugt im Bereich von 1:100 bis 1.000:1, besonders bevorzugt im Bereich 2:1 bis 1.000:1 liegen.

Makroskopische Oberflächen können gleichfalls nach den aus dem Stand der Technik bekannten Methoden mit den Silylpolyethern 1 beschichtet werden. Dabei können die Silylpolyether 1 entweder in Reinform oder aber in Abmischung mit weiteren Komponenten, z. B. anorganischen und/oder organischen Lösemitteln, reaktiven Komponenten wie mono-, oligo- oder polymeren Silanen, Acrylaten, Epoxiden, Hydroxyverbindungen, Aminen u.a.m. sowie weiteren Beschichtungskomponenten oder Hilfsstoffen, zur Oberflächenmodifizierung eingesetzt werden.

Die Applikation der Silylpolyether 1 kann dabei in Reinform pur, in organischen oder anorganischen Lösemitteln, als wässerige Emulsionen, als Mischungen der Silylpolyether 1 mit monomeren Silanen der Formel (2) und/oder anderen Silylgruppen tragenden Polymeren, in Kombination mit andersartig funktionalisierten Modifizierungsmitteln wie beispielsweise Epoxiden, Acrylaten, Aminen und/oder anderen Polymeren erfolgen.

Die Modifizierung makroskopischer Oberflächen mit den beschriebenen Materialien kann beispielsweise mit den aus dem Stand der Technik bekannten Verfahren wie Tauch-, Spray- oder Spin-Beschichtung, Fluten, Vernebeln, Aufpinseln, Aufrollen, Aufdrucken, Siebdrucken, Aufstempeln und - bei geeigneter Konsistenz der zur Oberflächenmodifizierung verwendeten erfindungsgemäßen Rezepturen - auch durch Pulverbeschichtungsverfahren durchgeführt werden. Weiterhin ist es auch möglich, Emulsionen aus den Silylpolyethern 1 in geeigneten organischen und/oder anorganischen Lösemitteln, ggf. unter Zusatz weiterer Stoffe wie z. B. Beschichtungskomponenten, Hilfsstoffen, wie beispielsweise Netzmitteln, Emulgatoren und/oder Rheologieadditiven, sowie Füllstoffe und/oder funktioneller Partikel, zur Modifizierung der Oberflächen zu verwenden.

Beispiele derartiger Oberflächen sind makroskopische und mikroskopische Oberflächen wie Oberflächen aus Glas, Lacken, Metallen, Halbleitermaterialien, oxidischen Materialien wie Steinen, Betonen oder Mörteln, Holz, organischen und anorganischen Fasern, Geweben und Partikeln, Polymeren, Biopolymeren u. a. m.

Als Katalysatoren für die chemische Fixierung der Silylpolyether 1 auf Partikel- und makroskopischen Oberfläche können die bekannten Polyurethanisierungs-, Allophanatisierungs- oder Biuretisierungskatalysatoren verwendet werden, die dem Fachmann an sich bekannt sind, bzw. die literaturbekannten und üblicherweise zur Hydrolyse und Kondensation von Alkoxysilanen eingesetzten Katalysatoren. Hierzu zählen Verbindungen, wie beispielsweise die Zinksalze Zinkoctoat, Zinkacetylacetonat und Zink-(II)-ethylcaproat, oder Tetraalkylammoniumverbindungen, wie N,N,N-Trimethyl-N-2-hydroxypropylammoniumhydroxid, N,N,N-Trimethyl-N-2-hydroxypropylammonium-2-ethylhexanoat oder Cholin-2-ethylhexanoat verwendet werden. Bevorzugt ist die Verwendung von Zinkoctoat (Zink-(II)-ethylhexanoat) und der Tetraalkylammoniumverbindungen, besonders bevorzugt diejenige von Zinkoctoat. Weiterhin können als Katalysatoren die üblicherweise verwendeten organischen Zinnverbindungen, wie z.B. Dibutylzinndilaurat, Dioctylzinndilaurat, Dibutylzinndiacetylacetonat, Dibutylzinndiacetat oder Dibutylzinndioctoat etc., verwendet werden. Des Weiteren können auch Bismutkatalysatoren, z.B. der Bor-chi-Katalysator, Titanverbindungen, z.B. Titan(IV)isopropylat oder Titanylacetylacetonat, Eisen(III)-Verbindungen, z.B. Eisen(III)acetylacetonat, Aluminiumverbindungen, wie Aluminiumtriisopropylat, Aluminiumtri-sec-butylat und andere Alkoholate sowie Aluminiumacetylacetonat, Calciumverbindungen, wie Calciumdinatriumethylendiamintetraacetat oder Caliumdiacetylacetonat, oder auch Amine, z.B. Triethylamin, Tributyla-min, 1,4-Diazabicyclo[2,2,2]octan, 1,8-Diazabicyclo[5.4.0]undec-7-en, 1,5-Diazabicyclo[4.3.0]non-5-en, N,N-Bis-(N,N-dimethyl-2-aminoethyl)-methylamin, N,N-Dimethylcyclohexylamin, N,N-Dimethylphenylamin, N-Ethylmorpholin etc., eingesetzt werden. Auch organische oder anorganische Brönstedsäuren wie Essigsäure, Trifluoressigsäure, Methansulfonsäure, p-Toluolsulfonsäure oder Benzoylchlorid, Salzsäure, Phosphorsäure deren Mono- und/oder Diester, wie z.B. Butylphosphat, (Iso-)-Propylphosphat, Dibutylphosphat etc., sind als Katalysatoren geeignet. Selbstverständlich können auch Kombinationen mehrerer Katalysatoren eingesetzt werden.

Die erfindungsgemäßen Zusammensetzungen enthaltend die härtbaren Modifizierungsmittel können auch sogenannte photolatente Basen als Katalysatoren enthalten, wie sie in der WO 2005/100482 beschrieben sind. Unter photolatenten Basen sind vorzugsweise organische Basen mit einem oder mehreren basischen Stickstoffatomen zu verstehen, die zunächst in einer blockierten Form vorliegen und erst nach Bestrahlung mit UV-Licht, sichtbarem Licht oder IR-Strahlung durch Spaltung des Moleküls die basische Form freisetzen.
Der Katalysator bzw. die photolatente Base wird in Mengen von 0,001 bis 10,0 Gew.-%, bevorzugt 0,01 bis 1,0 Gew.-% und besonders bevorzugt 0,05 bis 0,5 Gew.-% bezogen auf die alkoxysilylfunktionellen Silylpolyether 1 eingesetzt. Der Katalysator bzw. die photolatente Base kann in einer Portion oder aber auch portionsweise oder auch kontinuierlich zugegeben werden. Bevorzugt ist Zugabe der gesamten Menge in einer Portion.
Als weitere Komponenten können die Mischungen vorzugsweise weitere zumeist monomere Silane Hydroxylgruppen-tragende Siloxane oder Lösungsmittel enthalten.
Als weitere Silane können dabei prinzipiell sämtliche Silane, bevorzugt mit hydrolysierbaren Alkoxygruppen, eingesetzt werden und insbesondere solche Verbindungen wie in Formel (2) sowie diejenigen, die in der DE 10 2006 054155 oder der WO 2005/003201 beschrieben sind.
Des Weiteren können die Mischungen auch an sich bekannte Hilfsstoffe, wie von den Komponenten abweichende Wasserfänger, weitere Haftvermittler und/oder Reaktivverdünner sowie Weichmacher, beispielsweise Phthalate, Benzoate, Phosphatweichmacher, Thixotropiermittel, Fungizide, Flammschutzmittel, Pigmente etc. enthalten. Auch Lichtschutzmittel, Antioxidantien, Radikalfänger sowie weitere Stabilisatoren können den Mischungen zugesetzt werden.
Die Mischungen können organische Substanzen, bevorzugt Flüssigkeiten und Lösungsmittel enthalten. Die Lösungsmittel dienen dabei beispielsweise der Erniedrigung der Viskosität der unvernetzten Mischungen und begünstigen das Aufziehen auf die Partikeloberfläche. Als Lösungsmittel kommen prinzipiell sämtliche Lösungsmittel sowie Lösemittelmischungen in Betracht. Bevorzugte Beispiele für solche Lösungsmittel sind Ether wie z.B. t-Butyl-methylether, Ester, wie z.B. Ethylacetat oder Butylacetat oder Diethylcarbonat sowie Alkohole, wie z.B. Methanol, Ethanol sowie die verschiedenen Regioisomere des Propanols und Butanols oder auch anwendungsspezifisch ausgewählte Glykoltypen. Weiterhin können aromatische und/oder aliphatische Lösemittel wie auch halogenierte Lösemittel, wie z.B. Dichlormethan, Chloroform, Tetrachlormethan, Fluorkohlenwasserstoffe (FREON) u.a.m zum Einsatz kommen, aber auch anorganische Lösemittel wie beispielsweise Wasser, CS₂, überkritisches CO₂ u.a.m..

Die Anwendungsmöglichkeiten derart modifizierter Oberflächen oder Partikeloberflächen sind vielfältig. So können derart behandelte Partikel beispielsweise als Füllstoffe für Polymere bzw. die Herstellung von Polymercompounds, Nanokomposite und Masterbatches verwendet werden. Eine gute Übersicht über funktionale Füllstoffe in Polymeren bietet "Functional Fillers for Plastics", Edited by Prof. Dr. Marino Xanthos, WILEY-VCH Verlag GmbH & Co. KgaA, Weinheim, 2005, ISBN 3-527-31054-1. Die Verwendung der erfindungsgemäßen Silylpolyether kann dabei dergestalt sein, dass man entweder die zu modifizierenden Partikel in einem vorangeschalteten Prozess modifiziert und dann im Polymer dispergiert, ebenso ist es aber auch möglich, die Silylpolyether 1 bei der Dispergierung der Füllstoffe im jeweiligen Polymer zuzusetzen, beispielsweise über eine Flüssigdosierung im Extruder unter Nachschaltung einer effektiven Dispergierstrecke. Überraschenderweise gelingt i. d. R. die Partikeloberflächenmodifizierung mit den Silylpolyethern 1 ohne ein Verklumpen oder Aggregieren der zu modifizierenden partikulären Materialien trotz des multifunktionellen Charakters der Silylpolyether 1. Weiterhin können erfindungsgemäß oberflächenmodifizierte Partikel beispielsweise eingesetzt werden als Füllstoffe oder funktionale Additive in Lacken, polymeren Schäumen, organischen Harzen oder Silikonharzen, ggf. unter reaktiver Anbindung an die jeweiligen Matrices, als Melt-Flow-Index-Improver in Spritzgussanwendungen, zur Erzielung physikalischer Effekte auf Oberflächen, wie z. B. Superhydrophobie, temperaturabhängiger Benetzbarkeit, Abperleffekten, Beeinflussung des Anschmutzungsverhaltens und des Schmutzentfernungsverhaltens an festen Oberflächen auf Bauten, Textilien oder Fasern sowie der Anhaftung von Kondensaten und Eis an mit den erfindungsgemäßen beschichteten ausgerüsteten Oberflächen und Partikeln, als Gleitadditive bzw. Schmiermittel, in Versiegelungen, zur Erzielung von haptischen Effekten, wie beispielsweise eines seidigen Griffes (Soft-Touch-Oberflächen) oder einer bestimmten Oberflächengriffigkeit oder -rauhigkeit (Grip), als Mattierungsmittel, als Anbindungsstellen für weitere Materialien, wie beispielsweise andere Beschichtungsmaterialien, als Ad- oder Absorbentien in beispielsweise Papier- oder Filtermaterialien oder Stoffen, als selbstdispergierbare Partikel zur Herstellung von Dispersionen, als partikuläre Emulgatoren (für sog. "Pickering-Emulsionen" (s. a. "Emulsions", Spencer Umfreville Pickering, Journal of the Chemical Society, Transactions (1907), 91, 2001-2021), als reaktive und/oder vernetzbare Partikel, ggf. dispergiert in flüssigen Medien, als wirksame Komponenten in Entschäumern, in Bautenschutzmitteln, beispielsweise als aktive Komponenten zur integralen Massenhydrophobierung, als strukturierte, hydrophobe Komponente zur Oberflächenhydrophobierung oder als Träger für aktive, flüssige Komponenten, als (ggf. reaktive) Verkapselungsmittel, wie z. B. für Core-Shell-Partikel oder zur Mikroverkapselung flüssiger Systeme, zur Modifizierung von Membranmaterialien, bspw. zur Erzielung einer bestimmten, einstellbaren Porosität, Selektivität oder Permeabilität, als antistatische Additive, beispielsweise nach einer hydrophilen oder hygroskopischen Partikeloberflächenmodifizierung, als Rieselhilfsmittel, als Additive zur Erzielung oder Verbesserung der Kratzfestigkeit der mit den Partikeln ausgestatteten Materialien oder Oberflächen oder als partikuläre Additive mit weiteren Funktionen, beispielsweise als mikrobizide Additive, als Fluoreszenzmarker oder als Effektpigmente, als Trennmittel, als Bestandteile für Tieftemperaturbeständige Kabelbeschichtungen, als Herstellungskomponenten von Gummiteilen und Membranen, als Schlichte oder Bestandteile für Schlichten in der Textil- und Glasfaserindustrie, für Papier, als Additive für Toner, als Abrasiva oder Faltenkaschierungsmittel in Kosmetika, als über einen längeren Zeitraum hinweg Wirkstoffe oder Hilfsstoffe freisetzende Formulierungsbestandteile bzw. Trägermaterialien, wobei als freizusetzende Substanzen beispielsweise kosmetische Öle und Wirkstoffe, Duftstoffe, pharmazeutische Wirkstoffe, antimikrobielle Wirkstoffe, auch zum Beispiel Silber und silberhaltige Verbindungen, sowie Farb- und Konservierungsstoffe in den Partikeln vorhanden sein können, u.a.m..
Ein weiterer Gegenstand der Erfindung ist somit die unter Verwendung der Zusammensetzungen enthaltend die Silylpolyether (1), (5) und/oder (6) hergestellten, oben genannten beschichteten Flächen und Stoffe, Materialien.

Diese reaktiven Alkoxysilylgruppen tragenden Polyether der Formel (1) können zur Oberflächenmodifizierung oder als Beschichtung, als Bulkmaterial sowie auch, beispielsweise durch eine Suspensionspolymerisation, zur Herstellung partikulärer Materialien verwendet werden. Deren Vernetzung zu festen duroplastischen Endprodukten und Fixierung auf reaktive Gruppen, insbesondere Hydroxylgruppen aufweisenden Untergründen, erfolgt auf einfache Weise in Gegenwart von Wasser und wahlweise unter Zusatz von Säure oder Base oder anderer Beschleuniger, wobei durch Erhöhung der Temperatur während des Härtungsvorgangs die Härtungsgeschwindigkeit gesteuert werden kann. Der Polymeraufbau dieser vernetzbaren Polyether kann je nach und Art des Starters sowie nach Art, Menge und Abfolge der einsetzbaren Epoxidmonomere auf mannigfaltige Weise variiert werden, um auf diesem Weg wichtige anwendungstechnische Produkteigenschaften abhängig vom jeweiligen Verwendungszweck maßzuschneidern. So lässt sich beispielsweise durch eine Variation des Anteils an Alkoxysilan-Einheiten in der Polymerkette die Vernetzungsdichte und damit das mechanische und physiko-chemische Eigenschaftsprofil der ausgehärteten Polymere und der damit ausgerüsteten Oberflächen bzw. der Partikel, auf deren Oberfläche sie verankert sind, in weiten Grenzen beeinflussen. Überraschenderweise sind hier selbst mit beachtlicher Alkoxysilyl-Funktionalisierungsdichte ausgestattete Silylpolyether 1 niedrigviskose, gut handhabbare Flüssigkeiten, so dass auch im Falle angestrebter hochvernetzter, gut haftender Beschichtungen keinerlei Einschränkungen im Hinblick auf die Dosierung dieser Komponente zu verzeichnen sind.

Diese Beobachtung differenziert die erfindungsgemäße Lehre von der in der DE 10 2006 054155 dargelegten Vorgehensweise, die auf das Einbringen freier Silanmonomeren als Formulierungsbestandteile in den Endrezepturen abstellt, um zu gewährleisten, dass die notwendige Vernetzungsdichte bei gleichzeitig niedriger Verarbeitungsviskosität erzielt wird. Die im Hinblick auf ihre strukturelle Vielfalt kaum einzugrenzenden Alkoxysilylgruppen aufweisenden Silylpolyether 1 eröffnen dem in der Polymerchemie vertrauten Fachmann durch den Einbau z.B. von Ester-, Carbonat- und aromatischen Strukturelementen eine Gestaltungsfreiheit, die nahezu beliebige anwendungstechnische Bedürfnisse adressiert.

Die in der noch nicht vorveröffentlichen Schrift DE 10 2008 000360.3 beschriebenen Silylpolyether 1, und bevorzugt solche mehr als eine Silylgruppe pro Hydroxylgruppe tragende Verbindungen, eignen sich zur besonders gleichmäßigen und fest anhaftenden Oberflächenmodifizierung von anorganischen und organischen Oberflächen, Partikeloberflächen und/oder Porenoberflächen. Ein weiterer Vorteil kann sein, dass es unter Selbstvernetzung der Präpolymere zur Vergelung bzw. Verbrückung kommt.
Möglich wird dies u.a. dank der niedrigen Viskosität der Silylpolyether 1 als Alkoxysilyl-Präpolymere und deren hoher Reaktivität gegenüber den zu modifizierenden Oberflächen.
Dies gestattet es, unterschiedlichste Oberflächen, bestehend zum Beispiel aus Metalloxiden, Mischoxiden, Nitriden, Hydroxiden, Carbiden, Carbonaten, Silikaten, Pigmenten, Rußen, Elementen oder Legierungen sowie auch Oberflächen aus organischen Materialien zu modifizieren. Darüber hinaus sind natürlich auch die Oberflächen organischer Partikel, wie solcher aus Siliconharzen, organomodifizierten Siliconen, organischen Polymeren oder Biopolymeren, einer Oberflächenmodifizierung zugänglich.

Damit können die Silylpolyether 1 beispielsweise als Grundstoffe für die Herstellung von Klebstoffen, als reaktive Vernetzer, als Haftvermittler und Primer sowie Bindemittel für Metalle, Glas und Glasfasern/Glasgewebe, Holz, Holzwerkstoffen, Naturfasern, zur Ausrüstung und Behandlung von gegebenenfalls textilen Flächengebilden und Fasern aus natürlichen und/oder synthetischen sowie mineralischen Rohstoffen sowie beispielsweise auch Kork, Leder, Papier, Tissue, silikatischen und oxidischen Materialien dienen.
Der gezielte Einbau der sich über hydrolytische Prozesse an Mauerwerk, Beton, Mörtel, usw. verankernden Alkoxysilylgruppierungen erweist sich als äußert vorteilhaft bei Einsatz derartig ausgerüsteter Systeme im Bereich der Bauwirtschaft, wo es um die verbindende und isolierende Abdichtung von z.B. Zargen für Fenstern und Türen in Rohbauten geht.
Ein weiterer Gegenstand der Erfindung ist die Bautenhydrophobierung durch Verwendung von Silylpolyether 1 enthaltenden Hydrophobierungsmitteln und die damit je nach Anwendungszweck gewährleistete und gezielt einstellbare Hydrophobie der Oberfläche, die einerseits eine Durchnässung der Oberfläche vermeiden hilft, andererseits aber Wasserdampf passieren lässt.
Insbesondere bei Polyethern der Formel 1 mit einem hydrophoben Rest oder aber hohen PO, BO oder SO-Gehalt (Gehalt von Propylenoxid, Butylenoixd und/oder Styroloxid) lassen sich hohe Hydrophobierungsraten erzielen. Insbesondere SO-haltige Polyether sind z. B. gut geeignet, Kohlenstoffmaterialien, wie z. B. Graphit und/oder Ruß zu modifizieren, so dass diese z.B. einfacher dispergieren sind. Die Ausrüstung oder Behandlung der Flächengebilde dient einerseits dem Schutz der Oberfläche oder Faser, deren Eigenschaftsverbesserung und/oder Eigenschaftsveränderungen oder der Erzielung neuer Eigenschaftsprofile.
So können beispielsweise Graphit und auch hexagonales Bornitrid in einen Silylpolyether 1 eingearbeitet haben, um sogenannte Gleitlacke herzustellen. Diese Gleitlacke weisen als haptischen Effekt "Glätte" auf, im Unterschied zu beispielsweise mit AEROSIL^{®} 200 gefüllten Beschichtungen, die sehr "griffig" erscheinen.
Ein weiterer Gegenstand der Erfindung sind die so hergestellten tribologische und/oder haptischen Beschichtungen enthaltend Graphit bzw. Bornitrid.

Ein weiterer Gegenstand der Erfindung sind Dichtungsmassen und/oder Klebemassen enthaltend die Silylpolyether 1, wobei auch eine Oberflächenbeschichtungen selbst bereits abzudichten oder zu kleben vermag. Diese Dichtungs- und/oder Klebemassen können insbesondere Gleitadditive wie beispielsweise auch MoS₂ oder PTFE-Partikel enthalten.

Weiterhin können die Silylpolyether 1 auch bei der Herstellung von elektrischen und/oder elektronischen Bauteilen wie beispielsweise auch OLEDs, Solarpanels Verwendung finden. Dabei können als Additive leitfähige Partikel oder ionische Flüssigkeiten enthalten sein und damit den Einsatz in leitfähigen Beschichtungen und leitfähigen Klebern, beispielsweise in Leiterbahnen, zur Kontaktierung und/oder antistatischen Ausrüstung, ermöglichen.

Die Silylpolyether 1 können alleine oder als Additive in wässrigen Systemen zur Behandlung der genannten Flächengebilde und Fasern genutzt werden und ermöglichen damit den Einsatz der so ausgerüsteten Flächengebilde und Fasern im Hygiene-, Medizin-, Bau-, Automobil-, Heimtextil-, Bekleidungstextil-, Sport- und Agrarbereich.

Den so oberflächenmodifizierten Partikeln oder Flächengebinden kommen damit neue oder optimierte Eigenschaften zu, wie beispielsweise in Bezug auf Weichheit, Gleiten, Wassertransport/-aufnahme, Wasser- /Ölabweisung, UV-Schutz, Selbstreinigung (Lotus Effekt) für z.B. Markisen, Flammschutz, Erhöhung der Festigkeit bei höchstmöglicher Flexibilität, Antistatik, Resistenz gegen Bakterien, Viren, Chemikalien.

Ein weiterer Gegenstand der Erfindung ist der Einsatz der Silylpolyether 1-Formulierungen beispielsweise auch in kosmetischen Anwendungen als Additive in Lack- oder Nagellackformulierungen.
Ein moderner Nagellack oder eine Nagelbeschichtungszusammensetzung dient dazu, eine ansprechende Form und Färbung von Finger- und Fußnägeln bereitzustellen. Zusätzlich wird der Nagel gegen Umwelteinflüsse geschützt und eine Härtung der Nagelplatte oder der Nageloberfläche gewährleistet. Besondere Anstrengungen werden unternommen um Nagellackbeschichtungen langlebig, kratz- und splitterunempfindlich, glänzend in attraktiven Farben und Brillanz bereitzustellen. Nagellacke beinhalten (daher) eine große Anzahl verschiedenster Inhaltsstoffe, von denen die besonders wichtigen Filmbilder, Haftvermittler, Weichmacher, Lösungsmittel und Pigmente sind. Pyrogenes Silica wird als Rheologie und Thixotropie-Modifizierer verwendet. US Pat No. 4,873,077, GB 1177420 und DE 69111621 beschreiben eine Vielzahl von Additiven um einen guten Tragewiderstand, gute Splittervermeidung, das Brechen und Einreißen der Nägel und die Langlebigkeit nach dem Trocknen des Nagellacks als flexibler, gut haftender harter Film auf dem Nagel zu gewährleisten.

Weiterhin ist es mit den Silylpolyethern 1 auch möglich, gezielt physikalische Effekte auf festen Substraten hervorzurufen, wie beispielsweise hydrophobe oder hydrophile Oberflächeneigenschaften. Dabei können derartige Effekte weiterhin auch noch einem zusätzlichen Stimulus unterliegen, wie beispielsweise der herrschenden Temperatur. Literaturbekannt weisen Polyether in Wasser temperaturabhängig sogenannte Trübungspunkte auf, die aus der Inkompatibilisierung zum umgebenden Medium bei steigender Temperatur resultieren. Es konnte gezeigt werden, dass es gelingt, über die Anbindung silylmodifizierter Polyetherketten an unterschiedlichen Oberflächen deren Kontaktwinkel gegenüber verschiedenen Flüssigkeiten, beispielsweise Wasser, temperaturabhängig zu gestalten.

Im Rahmen zunehmenden Umweltbewusstseins ist die Zugabe von organischen Lösemitteln zur Erniedrigung der Viskosität von Formulierungen zur Modifizierung von Oberflächen in den letzten Jahren jedoch zunehmend in die Kritik geraten. Als Alternative bietet es sich an, die erfindungsgemäßen Präpolymere in der Form einer, vorteilhaft wässrigen, Emulsion zu applizieren. Emulsionen enthaltend silylfunktionalisierte Präpolymere sind in der Literatur beschrieben worden. In der Schrift DE 2558653 beschreibt Chang Emulsionen aus selbstemulgierenden, Silylgruppen tragenden Polyurethanen und deren Anwendung für die Beschichtung von Oberflächen. In der Schrift US 4,376,149 beschreibt Martin emulgierte Gemische aus kettenendständig silylierten Polyethern und OH-Siloxanen und deren Einsatz für die Beschichtung von Textilien. Klauck, Maier und Berthauer beschreiben in der Schrift DE 4215648 lagerstabile Kontaktklebstoffe auf Basis von Lösungen beziehungsweise Emulsionen von kationisch modifizierten, alkoxysilanterminierten Polyurethanen. US 6,713,558 und US 6,831,128 beschreiben wasserverdünnbare Emulsionen von silylierten Elastomeren und deren Herstellung, WO 2007/072189 und WO 2008/090458 Emulsionen aus Silylgruppen tragenden Polymeren.
Eine weitere Anwendung der Alkoxysilylgruppen tragenden Verbindungen der Formel (1) sind wässerige Emulsionen. Als Emulgatoren für solche Emulsionen kommen prinzipiell sämtliche anionischen, nicht-ionischen, kationischen und amphoteren Emulgatoren sowie Emulgatormischungen in Betracht. Bevorzugte Beispiele von solchen Emulgatoren sind Alkoholethoxylate, Fettsäureethoxylate, ethoxylierte Ester, und (ethoxylierte) Sorbitanester.
Die wässrige Phase der Emulsionen kann hydrophile, anorganische Füllstoffe zur Modifizierung der mechanischen Eigenschaften der erfindungsgemäßen Beschichtungen enthalten mit der Maßgabe, dass diese hydrophile Füllstoffe nachträglich zu der bereits stabilisierten Emulsion zugegeben werden. Vorteilhaft kann es sein, wenn die Oberfläche der eingesetzten Füllstoffe mindestens eine funktionelle Gruppe aufweist, so dass es nach Eintrocknen beziehungsweise Brechen der Emulsion zu chemischen Reaktionen zwischen reaktiven funktionellen Gruppen des Silylpolyethers 1 mit denen auf der Füllstoffoberfläche kommt. Beispiele solcher Füllstoffe sind pyrogene und gefällte Kieselsäure, anorganische Oxide wie Aluminiumoxid, Titandioxid und Zirkondioxid, Glas und Quarz, Hydroxide wie Aluminium- und Magnesiumhydroxid, Silicate wie Wollastonit, Glimmer, Kaolin und Talk, Calciumcarbonat und andere Carbonate, Metalle wie Kupfer, Zink und Nickel und Metalllegierungen sowie Graphit und Ruß.
Des Weiteren kann die Emulsion niedrigmolekulare, organofunktionelle und wasserunlösliche Silane enthalten, wie vorher beschrieben. Die Emulsion kann ebenfalls die vorher beschriebenen Katalysatoren zur Fixierung der Silylpolyether 1 auf eine Oberfläche enthalten.
Ebenso können den Emulsionen weitere funktionelle Stoffe zugesetzt werden. Dazu zählen beispielsweise rheologische Additive, Entschäumer, Entlüfter, filmbildende Polymeren, antimikrobielle und konservierende Stoffe, Antioxidantien, Farbstoffe, Färbemittel und Pigmente, Frostschutzmittel, Fungizide, Haftvermittler und/oder Reaktivverdünner sowie Weichmacher und Komplexbildner.

Auch Sprühhilfsmittel, Netzmittel, Vitamine, Wuchsstoffe, Hormone, Duftstoffe, Lichtschutzmittel, Radikalfänger, UV-Absorber sowie weitere Stabilisatoren können den Mischungen zugesetzt werden.

Ein weiterer Gegenstand der Erfindung ist die Herstellung von flammhemmenden thermoplastischen Polymercompounds oder duroplastischen Formmassen enthaltenden Silylethern 1, die darüber hinaus flammschützende und/oder flammenhemmenden Substanzen wie beispielsweise ATH (Aluminiumtrihydrat = Aluminiumhydroxid = Aluminiumtrihydroxid), MDH (Magnesiumdihydroxid) oder Melamincyanurat, enthalten können. Derartige Polymercompounds werden bsp. Weise für die Herstellung von Kabelisolationsmaterialien auf Basis von Polypropylen, Polyethylen oder Ethylvinylacetat für Kabel und Kabelummantelungen eingesetzt oder es werden z.B. auf Basis von Polypropylen flammgeschützte Trennwände hergestellt, die in öffentlichen Gebäuden wie z.B. Sporthallen besonders hohen Anforderungen unterliegen.
Die so ausgerüsteten Flammschutzmassen, -compounds oder auch Elektrokabel weisen gegebenenfalls eine verbesserte mechanische Stabilität, verbesserte Dispergierung weiterer Additive, gute Extrudierbarkeit auch bei hohen Füllgrad mit partikulären Additiven (beispielsweise mit Talk, Calciumcarbonat, u.a.) sowie verbesserten Flammschutz oder eine geringere Rauchentwicklung bei starker Erhitzung auf. Insbesondere bei der Verwendung Siloxangruppen aufweisender Silylpolyether kann durch den Siliciumgehalt eine zusätzliche Stabilität im Brandfall gegeben sein, da nach Abbrand ein zusätzlich stabilisierend wirkender und brandhemmender Anteil an SiO₂ zurückbleibt. Außerdem wird bereits beim Abbrand zu einem früheren Zeitpunkt eine sogenannte Hautbildung forciert, die das weitere Ansteigen der Massetemperatur reduziert und damit das Voranschreiten des Brandes gehemmt, was z.B. bei Kabeln, die von einem Raum in den nächsten Raum führen, besonders relevant ist.
Ein weiterer Gegenstand der Erfindung sind Verbundwerkstoffe wie beispielsweise Wood-Plastic-Composites (WPC) die unter Verwendung der Siliylpolyether 1 hergestellt werden, WPC's sind thermoplastisch verarbeitbare Verbundwerkstoffe, die aus unterschiedlichen Anteilen von Holz, Kunststoffen und Additiven bestehen, und durch thermoplastische Formgebungsverfahren, wie z.B. Extrusion, Spritzguss oder Presstechniken, verarbeitet werden. Gegenüber Polypropylen-Maleinsäureanhydrid-gepfropften Copolymeren weisen die neuartigen Silylpolyether-Composites eine verbesserte Anbindung an die Holz- oder Fasergrundkörper dieser Werkstoffe auf. Die Silylpolyether 1 binden dabei an die Fasern auf Basis Holz, Kokos oder anderen natürlich verfügbaren Faserprodukten unter Gleichzeitiger Hydrophobierung dieser Oberfläche auf und garantieren dadurch eine reduzierte Trockenzeit der Holzfaserpellets (Energieeinsparung!). Im Gegensatz zu gewöhnlichen anorganischen Füllstoffen können hier bereits niedermolekulare Produkte eine sehr gute compatibilisierende Wirkung entfalten, denn bei schnellen Extrusionsprozessen sind sie deutlich schneller homogen in Sekunden zu verteilen als die PP-MAA-Polymere.
Ein weiterer Gegenstand der Erfindung sind Pulverlackhärter mit definierter Glycidylfunktionalität und verbesserter Verträglichkeit und/oder Haftung (Haftverbesserung) mit dem Untergrund, die auch durch eine verbesserte Haftung unterstützt wird und wodurch Korrosionsunterwanderung in Pulverlackfassadenanwendungen reduziert wird. Die Haftverbesserung ist insbesondere bei oxidischen oder silikatischen Oberflächen wie beispielsweise Mörtel, Estrich oder Zement von wesentlicher Bedeutung.

Ein weiterer Gegenstand der Erfindung sind Flüssigpasten, in denen man die Silylpolyether der Formel 1 anstelle beispielsweise eines gewöhnlichen Polyetherpolyols (PPG 1000), welches im Allgemeinen die zusätzliche Verwendung eines Dispergiermittels erfordert, alleinig verwendet, da der Silylpolyether der Formel 1 die Eigenschaften beider Stoffe auf sich vereinigt. Derartige Pasten, die als Farbmittel Pigmente enthalten oder ggf. zusätzlich Farbstoffe u.a. Additive enthalten können, werden für die Einfärbung von polyolbasierten Systemen wir z.B. PU-Schaumstoffen, thermoplastischen Urethanen oder ähnlichem verwendet.

Weitere Gegenstände der Erfindung ergeben sich aus den Ansprüchen, deren Offenbarungsgehalt vollumfänglich Bestandteil der Beschreibung ist.

Der Gegenstand der vorliegenden Erfindung wird an Hand der Figuren 1 bis 3 näher erläutert ohne darauf beschränkt zu sein.

In Figur 1 ist die Formel (1) dargestellt.

In Figur 2 sind die Kontaktwinkel gegenüber Wasser einer Beschichtung auf Basis von Polyether SP-2, sowohl mit als auch ohne Silastic Fluid DC4-2737 (Beispiel 4) aufgetragen.

In Figur 3 sind zur Illustration der reduzierten Schüttdichten einige Ergebnisse der Bestimmung der Schüttdichten aus Beispiel 9 dargestellt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Soweit nicht explizit anders angegeben, sind in dieser Beschreibung und den nachstehenden Beispielen alle Mengen- und Prozentangaben auf das Gewicht bezogen, alle Drücke 0,10 MPa (abs.) und alle Temperaturen 20°C.
Abkürzungen: PO = Propylenoxid, EO = Ethylenoxid

Als Maß für die Reaktivitäten der Silylpolyether 1 ist verschiedentlich die Klebfreizeit angegeben. Darunter ist diejenige Zeitspanne zu verstehen, die nach dem Ausbringen des Silylpolyethers 1 an die Luft vergeht, bis die Polymeroberfläche soweit gehärtet ist, dass nach einer Berührung dieser Oberfläche mit einem Bleistift oder dem Finger weder Polymermasse daran haften bleibt, noch eine Fadenbildung auftritt.

### Experimenteller Teil:

In den nachfolgenden Beispielen wurden die folgenden Trialkoxysilylgruppen enthaltenden Silylpolyether 1 eingesetzt, die gemäß der noch nicht offengelegten Schrift DE 10 2008 000360.3 nach dem Verfahrensprinzip der DMC-katalysierten Alkoxylierung von 3-Glycidyloxypropyltriethoxysilan (Dynasylan® GLYEO) der Evonik Degussa GmbH bzw. 3-Glycidyloxypropyltrimethoxysilan (Dynasylan® GLYMO) der Evonik Degussa GmbH hergestellt worden sind.

### Trialkoxysilylpolyether SP-1:

Niedermolekularer, Octanol-gestarteter, fast farbloser und niedrig viskoser blockartig aufgebauter Polyether der mittleren Molmasse von ca. 3000 g/mol und siebenfacher Trialkoxysilanfunktionalität.

Chemischer Aufbau gemäß Monomerendosage: 1-Octanol + 8 mol PO + 3,5 mol GLYEO + 8 mol PO + 3,5 mol GLYEO + 2 mol PO
Epoxidsauerstoffgehalt <0,05 %, OH-Zahl 19,5 mg KOH/g, Viskosität (25,0 °C): 0,19 Pa.s

### Trialkoxysilylpolyether SP-2:

Niedermolekularer, Octanol-gestarteter, fast farbloser und niedrig viskoser, weitgehend statistisch aufgebauter Polyether der mittleren Molmasse von ca. 3000 g/mol und siebenfacher Trialkoxysilanfunktionalität.
Chemischer Aufbau gemäß Monomerendosage: 1-Octanol + 8 mol PO + (7 mol GLYEO / 10 mol PO) Epoxidsauerstoffgehalt <0,05 %, M_{w} lt. GPC 2760 g/mol, Viskosität (25,0 °C): 0,15 Pa.s

### Trialkoxysilylpolyether SP-3:

Niedermolekularer, Octanol-gestarteter, fast farbloser und niedrig viskoser, weitgehend statistisch aufgebauter Polyether der mittleren Molmasse von ca. 3000 g/mol und vierfacher Trialkoxysilanfunktionalität. Chemischer Aufbau gemäß Monomerendosage:
1-Octanol + 2 mol PO + (4 mol GLYEO/26 mol PO/8,5 mol EO) + 2 mol PO
Epoxidsauerstoffgehalt <0,05 %, OH-Zahl 22,0 mg KOH/g, Viskosität (25,0 °C): 3,18 Pa.s

### Trialkoxysilylpolyether SP-4:

Niedermolekularer, Octanol-gestarteter, fast farbloser und niedrig viskoser, blockartig aufgebauter Polyether der mittleren Molmasse von ca. 3000 g/mol und siebenfacher Trialkoxysilanfunktionalität.

Chemischer Aufbau gemäß Monomerendosage:
1-Octanol + 2 mol PO + (3 mol PO/3 mol EO) + 3,5 mol GLYEO + (5 mol PO/3 mol EO) + 3,5 mol GLYEO + 2 mol PO Epoxidsauerstoffgehalt <0,05 %, OH-Zahl 20,0 mg KOH/g, Viskosität (25,0 °C): 0,16 Pa.s

### Trialkoxysilylpolyether SP-5:

Niedermolekularer, Octanol-gestarteter, fast farbloser und niedrig viskoser, blockartig aufgebauter Polyether der mittleren Molmasse von ca. 2400 g/mol und 1,5-facher Trialkoxysilanfunktionalität.
Chemischer Aufbau gemäß Monomerendosage: 1-Octanol + 30 mol PO + 1,5 mol GLYMO + 3 mol PO
Epoxidsauerstoffgehalt <0,05 %, M_{w} lt. GPC 2650 g/mol, OH-Zahl 22,0 mg KOH/g Viskosität (25,0 °C): 0,32 Pa.s

### Trialkoxysilylpolyether SP-6:

Niedermolekularer, Octanol-gestarteter, fast farbloser und niedrig viskoser, blockartig aufgebauter Polyether der mittleren Molmasse von ca. 3000 g/mol und sechsfacher Trialkoxysilanfunktionalität. Chemischer Aufbau gemäß Monomerendosage:
1-Octanol + 4 mol PO + 3 mol GLYEO + 10 mol PO + 3 mol GLYEO + 10 mol PO
Epoxidsauerstoffgehalt <0,05 %, OH-Zahl 24,0 mg KOH/g, Viskosität (25,0 °C): 0,16 Pa.s

### Trialkoxysilylpolyether SP-7:

Niedermolekularer, Octanol-gestarteter, fast farbloser und niedrig viskoser, blockartig aufgebauter Polyether der mittleren Molmasse von ca. 2200 g/mol und einfacher Trialkoxysilanfunktionalität.

Chemischer Aufbau gemäß Monomerendosage: 1-Octanol + 2 mol PO + 1 mol GLYMO + 30 mol PO Epoxidsauerstoffgehalt <0,05 %, OH-Zahl 27,5 mg KOH/g, Viskosität (25,0 °C): 0,32 Pa.s

### Trialkoxysilylpolyether SP-8:

Höhermolekularer, Polyetherol-gestarteter, fast farbloser, statistisch aufgebauter Polyether der mittleren Molmasse von ca. 8200 g/mol und achtfacher Trialkoxysilanfunktionalität.
Chemischer Aufbau gemäß Monomerendosage: Polypropylenglykolmonobutylether (im Mittel 520 g/mol) + (8 mol GLYEO/94 mol PO)
Epoxidsauerstoffgehalt <0,03 %, OH-Zahl 7,6 mg KOH/g, Viskosität (25,0 °C): 1,68 Pa.s

### Trialkoxysilylpolyether SP-9:

Niedermolekularer, 1-Octanol-gestarteter, fast farbloser Polyether der mittleren Molmasse von ca. 2300 g/mol und einfacher Trialkoxysilanfunktionalität. Chemischer Aufbau gemäß Monomerendosage: 1-Octanol + 1 mol GLYEO + (9 mol EO/27,5 mol PO) Epoxidsauerstoffgehalt <0,05 %, OH-2ahl 25,5 mg KOH/g, Viskosität (25,0 °C): 0,33 Pa.s

### Trialkoxysilylpolyether SP-10:

Niedermolekularer, 1-Octanol-gestarteter, fast farbloser Polyether der mittleren Molmasse von ca. 2400 g/mol und einfacher Trialkoxysilanfunktionalität.
Chemischer Aufbau gemäß Monomerendosage:
1-Octanol + 2 mol PO + 1 mol GLYMO + (9 mol EO/27,5 mol PO)
Epoxidsauerstoffgehalt <0,05 %, OH-Zahl 25,0 mg KOH/g, Viskosität (25,0 °C): 0,29 Pa.s

**Tabelle 1 - Beispiel 1: Herstellung einer Emulsion**

| **Stoffe** | **Menge / Gramm** |
|---|---|
| TEGO^{®} Alkanol TD12 | 9, 0 |
| Rewopal^{®} LA3 | 6, 0 |
| Polyether SP-1 | 100,0 |
| Wasser | 100,0 |

9,0 g TEGO Alkanol TD12 (Isotridecylalkohol, Polyoxyethylen-(12)-Ether, Evonik Goldschmidt GmbH), 3,0 g Rewopal LA3 (Laurylalkohol, Polyoxyethylen- (3) -Ether, Evonik Goldschmidt GmbH) und 22,5 g Wasser wurden in einem Doppelwandglasgefäß auf 60 °C aufgeheizt und mit einer Mizerscheibe bei 1000 U/min homogenisiert bis eine homogene, viskose Paste entstand. Unter Zuhilfenahme eines Tropftrichters wurde innerhalb von 30 Minuten der Polyether SP-1 tropfenweise in die Paste unter Rühren eingearbeitet. Die fertige Paste wurde 10 Minuten bei 1000 U/min gerührt. Danach wurde die Paste mit den restlichen 77,5 Gramm Wasser verdünnt. Es entstand eine niedrigviskose Emulsion. Die Größe der Tropfen wurde mittels dynamischer Lichtstreuung (Malvern HPPS mit 633 nm HeNe-Laser) gemessen. Auswertung der Korrelationsfunktion mit dem CONTIN-Algorithmus ergab eine monomodale Tropfengrößenverteilung mit einem durchschnittlichen Tropfenradius von 135 nm.

### Beispiel 2: Beschichtung (1)

Mittels einer Transferpipette (Brand) wurde 1,00 mL Dibutylzinndiacetylacetonat (DBTAA) zu 50 mL der in Beispiel 1 beschriebenen Emulsion zugegeben. Das DBTAA wurde unter Rühren mit einer Mizersscheibe bei 1000 U/min in der Emulsion dispergiert. Eine Glasplatte wurde gereinigt, mit Isopropanol gespült und getrocknet. Unter Zuhilfenahme einer Kastenrakel wurde eine Emulsionsschicht mit einer Dicke von 150 Mikrometer auf die gereinigte Glasplatte aufgezogen. Der aufgezogene Emulsionsfilm wurde bei Raumtemperatur getrocknet und ausgehärtet.

### Beispiel 3: Beschichtung (2)

Mittels einer Transferpipette (Brand) wurde 1,00 mL Dibutylzinndiacetylacetonat (DBTAA) zu 50 mL Polyether SP-1 gegeben. Das DBTAA wurde unter Rühren mit einer Mizerscheibe bei 1000 U/min in das Präpolymer eindispergiert. Eine Glasplatte wurde gereinigt, mit Isopropanol gespült und getrocknet. Unter Zuhilfenahme einer Kastenrakel wurde eine Präpolymerschicht mit einer Dicke von 150 Mikrometer auf die gereinigte Glasplatte aufgezogen. Das aufgezogene Präpolymer wurde bei Raumtemperatur ausgehärtet.

### Beispiel 4: Beschichtung (3) und Bestimmung Kontaktwinkel

Mittels einer Transferpipette (Brand) wurde 1,0 g Dibutylzinndiacetylacetonat (DBTAA) zu einer Mischung aus 40 g Polyether SP-2 und 10 g Silastic Fluid DC4-2737 gegeben. Die Mischung wurde unter Rühren mit einer Mizersscheibe bei 1000 U/min homogenisiert. Eine Glasplatte wurde gereinigt, mit Isopropanol gespült und getrocknet. Unter Zuhilfenahme einer Kastenrakel wurde die Mischung mit einer Dicke von 150 Mikrometer auf die gereinigte Glasplatte aufgezogen. Die aufgezogene Mischung wurde bei Raumtemperatur ausgehärtet. Es entstand eine klare Beschichtung.
Anschließend wurde der Kontaktwinkel der Oberflächenbeschichtung gegenüber Wasser mit Hilfe eines Kontaktwinkelmessgerätes vom Typ OCA35 der Firma DataPhysics Instruments GmbH bestimmt. Das Ergebnis ist in der Figur 2 dargestellt. Der Kontaktwinkel beider Beschichtungen gegenüber Wasser (d.h. Polyether SP-2 mit und ohne Silastic Fluid DC4-2737) nimmt mit der Zeit ab. Dies deutet auf eine Änderung der Morphologie und/oder Chemie der Oberfläche bei Kontakt mit Wasser hin.

### Beispiel 5: Bestimmung der Überlackierbarkeit

Die Überlackierbarkeit der Beschichtung aus Beispiel 3 wurde überprüft, zu diesem Zweck wurde eine rote Flexofarbe verwendet. Die Flexofarbe bestand aus 47 % Joncryl 2647 (BASF), 10 % Joncryl 61 (BASF), 38% Flexoverse Red RCD5704 (Sun Chemicals) und 5% Wasser. Mittels einer Rakelmaschine (RK Print Coat Instruments Ltd.) bei Geschwindigkeitstufe 4 und einer 12 µm Rollrakel wurde die Flexofarbe auf die ausgehärtete Beschichtung aus Beispiel 3 aufgezogen. Nach dem Trocknen resultierte eine homogene Beschichtung, ohne Krater oder sonstige Zeichen der Entnetzung.

### Beispiel 6: Bestimmung der Pendelhärte

Die Pendelhärte der Beschichtungen aus den Beispielen 3 und 4 wurde mit einem Pendeldämpfungsprüfgerät 299/399 der Firma Erichsen nach DIN 53157 bestimmt. Die Pendelhärte, oft auch als Pendeldämpfung bezeichnet, ist eine in der Lackindustrie gebräuchliche Messgröße für die Härte einer Beschichtung. Diese wird mittels Schwingungs dämpfung eines auf der BeschichtungsOberfläche aufgesetzten Pendels ermittelt, das auf zwei Stahlkugeln als Auflagefläche hin und her schwingt. Die Schwingungsenergie des Pendels wird dabei umso mehr "gedämpft", je geringer die Härte der auf einer ebenen Fläche aufgezogenen, trockenen Beschichtung ist. Ermittelt wird die Anzahl von Schwingungen. Die Ergebnisse sind in der nachfolgenden Tabelle 2 zusammengefasst.

**Tabelle 2:**

| Pendelhärte, Abriebfestigkeit und Glanz verschiedener Beschichtungen | | | | |
|---|---|---|---|---|
| Beschichtung | Pendelhärte Schwingungen | Anzahl Hübe | Glanz | |
| | | | 60° | 85° |
| Polyether SP-3 | 26 | 2 | 155 | 113 |
| Polyether SP-1 | 68 | 3 | 154 | 112 |
| Polyether SP-4 | 11 | 1 | 156 | 118 |
| Polyether SP-2 | 26 | 1 | 156 | 117 |
| Polyether SP-2 / Silastic, 80:20 | 68 | 1 | 126 | 103 |

### Beispiel 7: Bestimmung der Abriebfestigkeit

Die Abriebfestigkeit der Beschichtung aus den Beispielen 3 und 4 wurde mit einem CM-5 Crockmeter der Firma ATLAS Material Testing Technology GmbH getestet. Hier wurde an einen Stift ein Stück Scheuerstoff (SCOTCH-Brite CF-HP Handschleifpad S Ultra fein-grau der Firma 3M) geklebt, der Stift wurde nun hubartig über die zu testende Fläche geschoben. Der Reibwinkel betrug dabei 90°. Der Zeitpunktunkt, an dem eine sichtbare Beschädigung der Oberfläche zu erkennen war, war der Zeitpunkt an dem die Messung gestoppt wurde. Ermittelt wurde die Anzahl von Hüben. Die Ergebnisse sind in der vorangestellten Tabelle 2 zusammengefasst.

### Beispiel 8: Bestimmung des Glanzes

Von den Beschichtungen aus den Beispielen 3 und 4 wurde der Glanz mit einem REFO 3-D Reflektometer der Firma Dr. Lange GmbH getestet. Glanzwerte wurden sowohl bei 60° als auch bei 85° gemessen. Die Ergebnisse sind in der vorangestellten Tabelle 2 zusammengefasst.

### Beispiel 9: Nachbehandlung von Füllstoffen durch direkte Aufbringung auf die Füllstoffoberfläche

Es wurden 495 g der folgenden Füllstoffe in einen Henschel-Mischer gegeben und dann jeweils 5 g der nichterfindungsgemäßen und erfindungsgemäßen Verbindungen zugegeben, um die Füllstoffoberflächen zu hydrophobieren. Dazu wurde für 15 Minuten das Gerät mit einer Geschwindigkeit von 900 Upm betrieben, wobei es zu einer Erwärmung auf bis zu 70 °C kommen kann. Anschließend wurden die Füllstoffe in einen 100 ml Messzylinder gegeben, um die Schüttdichtenunterschiede zu bestimmen. Die Ergebnisse sind Tabelle 3 wiedergegeben:

**Tabelle 3:**

| **Füllstoff/Pigment** | **Oberflächenbeh.** | **Füllhöhe in ml** |
|---|---|---|
| **Talkum HTP 1** | ohne Zusatz | 15g ergeben 70 ml |
| | mit TP 6875 | 15g ergeben 70 ml |
| | mit Siliconöl 1000 | 15g ergeben 75 ml |
| | mit SP-5 | 15g ergeben 63 ml |
| | mit SP-6 | 15g ergeben 60 ml |
| | mit SP-7 | 15g ergeben 64 ml |
| | mit SP-8 | 15g ergeben 68ml |
| | mit SP-1 | 15g ergeben 66ml |
| **ATH Apyral 40 CD** | ohne Zusatz | 30g ergeben 70 ml |
| | mit TP 6875 | 30g ergeben 90ml |
| | mit SP-5 | 30g ergeben 85 ml |
| | mit SP-8 | 30g ergeben 84 ml |
| | mit SP-7 | 30g ergeben 82 ml |

Erstaunlicherweise wurde gefunden, dass die resultierenden Schüttdichten trotz Hydrophobierung mit den erfindungsgemäßen Substanzen nicht ansteigen bei der Verwendung in Kombination mit Talk bzw. nicht so stark ansteigen wie nichterfindungsgemäße Substanzen es für ATH tun. Als nichterfindungsgemäße Produkte zur Hydrophobierung sind dabei Siliconöl oder Alkylsiloxanen (TEGEOPREN^{®} TP 6875, Evonik Goldschmidt GmbH) im Vergleich betrachtet worden.

Damit ist es möglich Füllstoffe mit niedrigerer Schüttdichte (bei Siloverladungen relevant für Lagerraum und Entleerung) zu erzeugen oder mit den erfindungsgemäßen Verbindungen Füllstoffoberflächen zu gestalten, die eine Befüllung von Masterbatchen und Compounds mit höherer Gewichtskonstanz im Extrudat erlauben, da durch die geringere Schüttdichte weniger Brückenbildung im Einzugsbereich der Füllstoffe auftritt, z.B. relevant für Füllstoffcompounds mit 60-80% Füllgrad mit Füllstoff in verschiedenen Polymeren wie z.B. Polypropylen. Die durch die neuartige Hydrophobierung erzeugte niedrigere Schüttdichte ist für Compoundeure wirtschaftlich relevant.
Zur Illustration der reduzierten Schüttdichten siehe auch Figur 3.

### Beispiel 10: Erfindungsgemäße Dispergierung von Füllstoffen in einem Polymercompound auf Basis von Polypropylen, entsprechend einem separaten Zusatz der Silylverbindungen

Mit einem Leistritz-Extruder 27 mm (Zweischneckenextruder der Firma Leistritz) wurden mit 200 Upm die folgenden Füllstoffcompoundrezepturen hergestellt:

| | |
|---|---|
| Polypropylen PPH 9069 | 39,8% |
| Irganox 1010 | 0,2% |
| Luzenac 20 MO bzw. Millicarb OG | 60,0% |
| diese werden hier mit den die Polyether enthaltenden Rezepturen verglichen: der Formel 1 | |
| Polypropylen PPH 9069 | 37,8% |
| Irganox 1010 | 0,2% |
| Additiv (nicht erf.gemäß bzw. erf.gemäß) | 2,0% |
| Luzenac 20 MO bzw. Millicarb OG | 60,0% |

Als nicht erfindungsgemäße Produkte, die zur Dispergierung von Füllstoffen und Pigmenten verwendet werden, sind typischerweise im Einsatz Stearate, Wachse oder auch hochwertige Copolymere (TEGOMER P 121 und E 525). Die Compounds müssen hinsichtlich ihrer mechanischen Eigenschaften geprüft werden, um die Anbindung des Füllstoffs an die Polymermatrix beurteilen zu können und den Grad der Dispergierung, der sich durch die Hydrophobierung der Füllstoffoberflächen verbessert, klassifizieren zu können. Dazu wurden folgende Eigenschaften geprüft:
1. Schlagzähigkeit (SZ) und Kerbschlagzähigkeit (KSZ) in kJ/m2 nach Izod ISO-Norm
2. Streckdehnung in Prozent nach DIN 53455

Darüber hinaus wurde von den Compounds der sogenannte MFI (Melt Flow Index) nach DIN ISO 53735 bestimmt, wobei bei Polypropylencompounds die Menge in Gramm Compound pro 10 Minuten Durchfluss ermittelt wurde und die Temperatur dazu 230°C mit einem Auflagegewicht von 2,16 Kilogramm beträgt.

Die ermittelten Werte sind der folgenden Tabelle 4 zu entnehmen:

**Tabelle 4:**

| Millicarb OG (Calciumcarbonat) | | | | |
|---|---|---|---|---|
| Compound | SZ | KSZ | Eta | MFI (230°C/2,16kg) |
| | kJ/m2 | kJ/m2 | % | g/10 min |
| Kein Additiv | 11,18 | 1,44 | 0,7 | 13 |
| TEGOMER P 121 | 11,85 | 1,74 | 0,9 | 15 |
| SP-5 | 32,15 | 2,09 | 2,4 | 18 |
| SP-7 | 23,88 | 2,08 | 0,9 | 15 |
| SP-6 | 13,49 | 2,06 | 1,0 | 16 |

| Luzenac 20 MO (Talkum) | | | | |
|---|---|---|---|---|
| Kein Additiv | 5,42 | 1,21 | - | 8 |
| TEGOMER E 525 | 5,67 | 1,22 | - | 6 |
| TEGOMER P 121 | 5,87 | 1,22 | - | 10 |
| SP-5 | 6,09 | 1,34 | - | 14 |
| SP-6 | 6,05 | 1,35 | - | 15 |
| SP-7 | 6,46 | 1,33 | - | 14 |

Bei der näheren Betrachtung der Ergebnisse in den Compounds wird ersichtlich, dass die erfindungsgemäßen Produkte gegenüber den nichterfindungsgemäßen und heute dem höchsten Stand der Technik entsprechenden Produkte eine deutlich bessere Anbindung der Füllstoffe an die Polymermatrix aufweisen, denn die erfindungsgemäßen Produkte zeigen eine deutliche Verbesserung der Schlagfestigkeit und Kerbschlagfestigkeit ohne dabei einen Verlust in der Flexibilität des Compounds - angezeigt durch die Streckdehnung in Prozent zu erzeugen. Die erfindungsgemäßen Produkte erlauben eine deutlich höhere Wirtschaftlichkeit, denn der MFI ist stets höher als ohne Additiv oder mit nichterfindungsgemäßen Produkten. Für Extrusionsprozesse ist damit bis zum doppelten Durchsatz zu rechnen und eine deutlich verbesserte Energieeffizienz zu verzeichnen.

### Beispiel 11: Erfindungsgemäße Oberflächenmodifizierung von Talkum-Partikeln mit Silylpolyethern 1 und Nachweis der Anbindung durch thermogravimetische Analyse der Partikel (Verlust von Ethanol bei Anbindung)

### a) Modifizierung von Talkum IMIFABI HTP 1 mit 1 Gew.-% Polyether SP-8

In einem Henschel-Mischer wurden 198 g Talkum IMIFABI HPT 1 vorgelegt. Unter starker Durchmischung (1000 Upm) wurden 2 g Polyether SP-8 durch ein Septum langsam eingespritzt. Die Durchmischung wurde nach erfolgter Zugabe des Silylpolyethers für weitere 20 min fortgesetzt. Die Temperatur stieg dabei auf bis zu 75 °C an. Eine thermogravimetrische Analyse (RT bis 800 °C, 5 °C/min, Luft) ergab eine Massenabnahme von 0,76 Gew.-% im Temperaturintervall von 140 bis 330 °C (berechneter Wert für die Massenabnahme durch Oxidation: ∼0,89 Gew.-%).

### b) Modifizierung von Talkum IMIFABI HTP 1 mit 1 Gew.-% Polyether SP-1

In einem Henschel-Mischer wurden 198 g Talkum IMIFABI HTP 1 vorgelegt. Unter starker Durchmischung (1000 Upm) wurden 2 g Polyether SP-1 durch ein Septum langsam eingespritzt. Die Durchmischung wurde nach erfolgter Zugabe des Silylpolyethers für weitere 20 min fortgesetzt. Die Temperatur stieg dabei auf bis zu 75 °C an. Eine thermogravimetrische Analyse (RT bis 800 °C, 5 °C/min, Luft) ergab eine Massenabnahme von 0,64 Gew.-% im Temperaturintervall von 140 bis 330 °C (berechneter Wert für die Massenabnahme durch Oxidation: ∼0,74 Gew.-%).

In beiden Fällen lag der beobachtete Wert der Massenabnahme deutlich unter dem Wert des zur Oberflächenmodifizierung eingesetzten erfindungsgemäßen Silylpolyethers (1 Gew.-%), was auf die Abspaltung von Ethanol und die kovalente Anbindung des Silylpolyethers an das Substrat schließen lässt.

### Beispiel 12: Erfindungsgemäße Oberflächenmodifizierung von planaren SiO₂-Oberflächen mit Silylpolyethern 1 und Bestimmung des temperaturabhängigen Kontaktwinkels gegenüber Wasser

Als Substrate kamen vorbehandelte Si-Wafer zum Einsatz, die eine oxidische Oberfläche aufwiesen. Diese Oberfläche sollte reaktive Hydroxyl-Gruppen aufweisen, um die Anbindung eines erfindungsgemäßen Silylpolyethers zu ermöglichen. Dazu wurden zwei unterschiedliche Vorbehandlungs-Verfahren angewendet: a) Die Wafer-Stücke wurden bei 1000 °C für eine Stunde in Luft geglüht und über Nacht in verdünnter (5%-iger) wäßriger AmmoniakLösung aufbewahrt. Danach wurden die Proben mit demin. Wasser abgespült und bis zur weiteren Verwendung in demin. Wasser gelagert. B) Die Wafer-Stücke wurden für eine Minute in eine frisch bereitete Mischung aus konz. H₂SO₄ und 30%-igem H₂O₂ im Verhältnis 1:1 getaucht, mit demin. Wasser gespült und bis zur weiteren Verwendung in demin. Wasser gelagert.
Es ist bekannt, dass statistisch verteilte EO/PO-Polyether einen sogenannten Trübungspunkt besitzen, d. h. sie sind in Wasser nur bis zu einer bestimmten Temperatur klar löslich, darüber werden diese Lösungen durch Ausfallen des Polyethers trübe. Solche Polyetherstrukturen mit einer EO/PO-Verteilung von 25/75 repräsentieren die Polyether SP-9 und SP-10. Beide Polyether wurden über eine Hydrolyse-Kondensationsreaktion an die vorbehandelten Wafer-Oberflächen angekoppelt.
Von beiden Silylpolyethern wurden jeweils 1 Gew.-%ige Lösungen in wasserfreiem Ethanol hergestellt, die vorbehandelten Wafer wurden mit Ethanol abgespült und in die ethanolische Silylpolyetherlösung gestellt. Nach Stehen über Nacht wurden die Behandlungslösungen mit konz. HCl auf einen pH-Wert von 1-2 angesäuert, nach weiteren 3 h wurden die Lösungen noch mit demin. Wasser versetzt, so dass der Wassergehalt der Behandlungslösungen bei ca. 1 Gew.-% lag. Nach erneutem Stehen über Nacht wurden die beschichteten Wafer mit Wasser und anschließend Ethanol gewaschen und bei Raumtemperatur an der Luft getrocknet.
An den so beschichteten Substraten wurde der dynamische Kontaktwinkel gegenüber Wasser bei 20 und bei 50 °C an mehreren Stellen bestimmt. Aufgrund des Verhaltens eines entsprechenden Polyethers in Wasser bei diesen Temperaturen wurde erwartet, dass die höhere Temperatur eine hydrophobere Oberfläche der beschichteten Wafer ergeben sollte. Die Ergebnisse sind in der folgenden Tabelle 5 dargestellt:

**Tabelle 5:**

| Beschichtetes Substrat | Gemittelter Kontaktwinkel bei 20 °C | Gemittelter Kontaktwinkel bei 50 °C |
|---|---|---|
| Blindwert(geätzter Si-Wafer, nicht beschichtet) | 52° | 44° |
| Geätzter Si-Wafer, mit SP-9 beschichtet | 50° | 57° |
| Geätzter Si-Wafer, mit SP-10 beschichtet | 46° | 54° |
| Geglühter Si-Wafer, mit SP-9 beschichtet | 43° | 48° |

## Patentansprüche

1. Verwendung von einem oder mehreren gemischten härtbaren hydroxygruppenhaltigen Silylpolyethern mit mindestens einer nicht-terminalen Silylfunktion als Bestandteil von Zusammensetzungen als Modifizierungsmittel und/oder Beschichtungsmittel für Oberflächen.

2. Verwendung nach Anspruch 1, **dadurch gekennzeichnet, dass** Verbindungen der Formel (1), 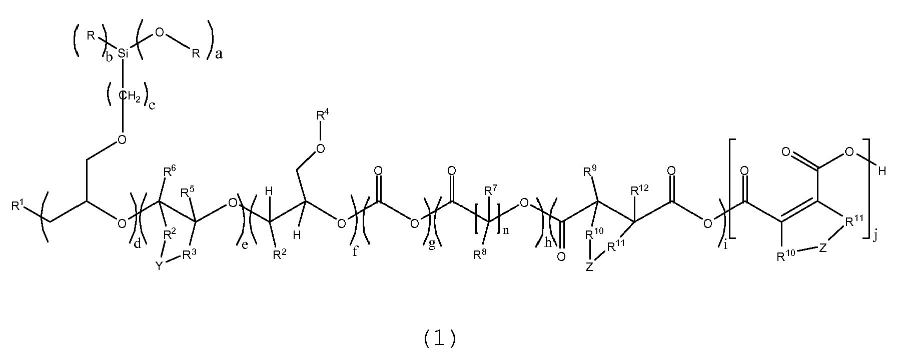 wobei
a eine ganze Zahl von 1 bis 3 ist,
b eine ganze Zahl von 0 bis 2 ist,
und die Summe von a und b gleich 3 ist,
c eine ganze Zahl von 0 bis 22 ist,
d eine ganze Zahl von größer 1 bis 1.000 ist,
e eine ganze Zahl von 0 bis 10.000 ist,
f eine ganze Zahl von 0 bis 1.000 ist,
g eine ganze Zahl von 0 bis 1.000 ist,
h, i und j ganze Zahlen von 0 bis 500 ist,
und mit der Maßgabe, dass die Fragmente mit den Indices d bis j untereinander frei permutierbar, d.h. in der Sequenz innerhalb der Polyetherkette gegeneinander austauschbar sind,
n eine ganze Zahl zwischen 2 und 8 ist und
R einen oder mehrere gleiche oder verschiedene Reste, ausgewählt aus linearen oder verzweigten, gesättigten, einfach oder mehrfach ungesättigten Alkylresten mit 1 bis 20 Kohlenstoffatomen oder Halogenalkylgruppen mit 1 bis 2 0 Kohlenstoffatomen darstellt,
und
R¹ gleich einem gesättigten oder ungesättigten, gegebenenfalls verzweigten Rest, der über ein Sauerstoffatom angebunden ist, oder einen Polyetherrest vom Typ einer Alkoxy-, Arylalkoxy- oder Alkylarylalkoxygruppe darstellt, bei der die Kohlenstoffkette durch Sauerstoffatome unterbrochen sein kann, oder eine ggf. einfach oder mehrfach annelierte aromatische Aryloxy-Gruppe ist, oder eine siliciumhaltige Verbindung oder ein Siloxanrest ist, der alkyl- und/oder arylgruppensubstituiert sein kann,
R² oder R³, sowie R⁵ oder R⁶ gleich oder auch unabhängig voneinander H oder ein gesättigter oder gegebenenfalls einfach oder mehrfach ungesättigter, auch weiter substituierter, gegebenenfalls ein- oder mehrwertiger Kohlenwasserstoffrest, wobei für die Reste R⁵ oder R⁶ gilt, dass sie gleich einem einwertigen Kohlenwasserstoffrest sind, wobei der Kohlenwasserstoffrest cycloaliphatisch über das Fragment Y verbrückt sein kann; Y kann nicht vorhanden sein, oder aber eine Methylenbrücke mit 1 oder 2 Methyleneinheiten sein, ist Y nicht vorhanden, so sind R² oder R³ unabhängig voneinander gleich ein linearer oder verzweigter Rest mit 1 bis 20 Kohlenstoffatomen,
R⁴ entspricht einem linearen oder verzweigten Alkylrest von 1 bis 24 Kohlenstoffatomen oder einem aromatischen oder cycloaliphatischen Rest, der gegebenenfalls seinerseits Alkylgruppen tragen kann,
R⁷ und R⁸ sind unabhängig voneinander entweder Wasserstoff, Alkyl-, Alkoxy-, Aryl- oder Aralkylgruppen, die unter Ringöffnungspolymerisation zu vernetzbaren, Alkoxysilangruppen enthaltenden Polyetherestern copolymerisiert werden,
R⁹, R¹⁰, R¹¹ und R¹² sind unabhängig voneinander entweder Wasserstoff, Alkyl-, Alkenyl-, Alkoxy-, Aryl- oder Aralkylgruppen wobei der Kohlenwasserstoffrest cycloaliphatisch oder aromatisch über das Fragment Z verbrückt sein kann und Z sowohl einen divalenten Alkylen- als auch Alkenylenrest darstellen kann, allein oder im Gemisch verwendet wird.

3. Verwendung nach Anspruch 2, **dadurch gekennzeichnet, dass** Silylpolyether enthalten sind, in denen die Summe der Indices (a) plus (b) in Formel (1) im statistischen Mittel kleiner als 3 ist.

4. Verwendung nach zumindest einem der Ansprüche 2 oder 3, **dadurch gekennzeichnet, dass** als Verbindungen der Formel (1) Verbindungen der Formel (5) oder deren Mischungen verwendet werden, wobei 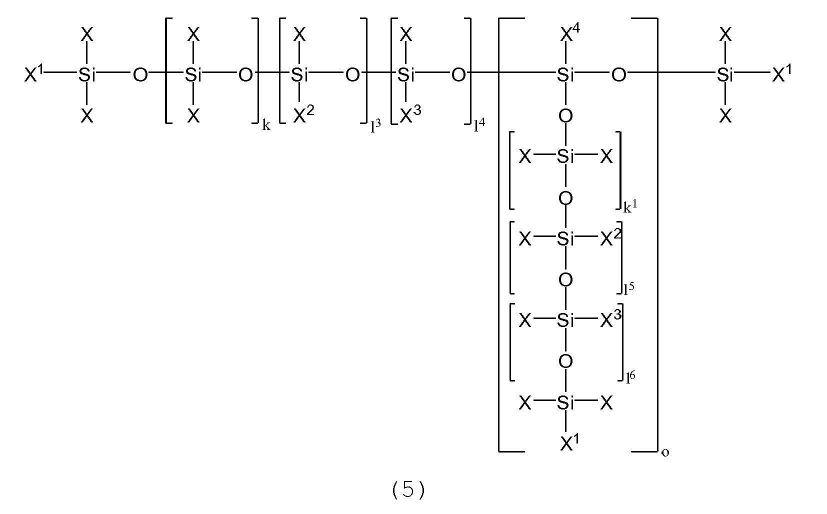 wobei
X ein linearer, cyclischer oder verzweigter, aliphatischer oder aromatischer, gesättigter oder ungesättigter Kohlenwasserstoffrest mit 1 bis 20 C-Atomen ist, der Heteroatome wie Sauerstoff, Stickstoff, Phosphor oder Schwefel enthalten kann,
X¹ wahlweise X, X² oder X³ ist,
X² ein Alkoxysilylgruppen tragender OH-funktioneller Polyoxyalkylenrest der Formel (5a) ist, der Ester- oder Carbonat-modifiziert sein kann, 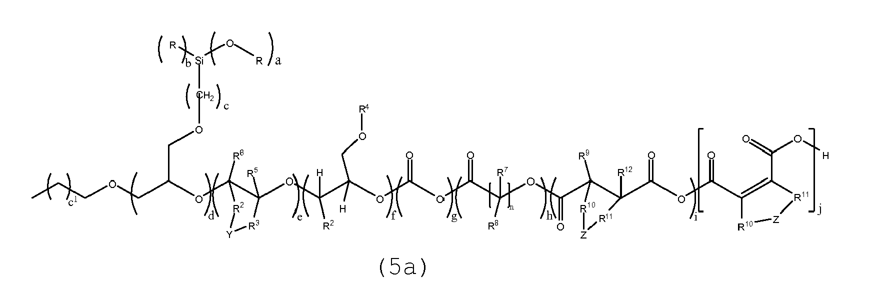
x³ ein endständig veretherter Polyoxyalkylenrest der Formel (5b) ist, 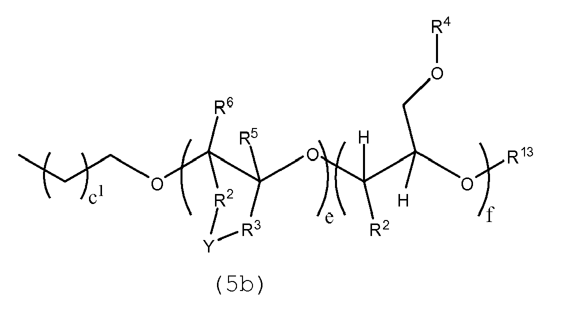 wobei
R stellt einen oder mehrere gleiche oder verschiedene Reste, ausgewählt aus linearen oder verzweigten, gesättigten, einfach oder mehrfach ungesättigten Alkylresten mit 1 bis 20 Kohlenstoffatomen oder Halogenalkylgruppen mit 1 bis 20 Kohlenstoffatomen dar,
R² oder R³, sowie R⁵ oder R⁶ sind gleich oder unabhängig voneinander H oder ein gesättigter oder gegebenenfalls einfach oder mehrfach ungesättigter, auch weiter substituierter, gegebenenfalls ein- oder mehrwertiger Kohlenwasserstoffrest, wobei für die Reste R⁵ oder R⁶ gilt, dass sie gleich einem einwertigen Kohlenwasserstoffrest sind und der Kohlenwasserstoffrest cycloaliphatisch über das Fragment Y verbrückt sein kann; Y kann nicht vorhanden sein, oder aber eine Methylenbrücke mit 1 oder 2 Methyleneinheiten sein; ist Y gleich 0, so sind R² oder R³ unabhängig voneinander gleich ein linearer oder verzweigter Rest mit 1 bis 20 Kohlenstoffatomen sein und die Kohlenwasserstoffreste R² und R³ können ihrerseits weiter substituiert sein und funktionelle Gruppen wie Halogene, Hydroxylgruppen oder Glycidyloxypropylgruppen tragen,
R⁴ ist ein linearer oder verzweigter Alkylrest von 1 bis 18 Kohlenstoffatomen, der an einen aromatischen oder cycloaliphatischen Rest gebunden sein kann,
R⁷ und R⁸ sind unabhängig voneinander entweder Wasserstoff, Alkyl-, Alkoxy-, Aryl- oder Aralkylgruppen,
R⁹, R¹⁰, R¹¹ und R¹² sind unabhängig voneinander entweder Wasserstoff, Alkyl-, Alkenyl-, Alkoxy-, Aryl- oder Aralkylgruppen, wobei der Kohlenwasserstoffrest cycloaliphatisch oder aromatisch über das Fragment Z verbrückt sein, wobei Z sowohl einen divalenten Alkylen- als auch Alkenylenrest darstellen kann,
R¹³ ist wahlweise eine Alkylgruppe mit 1 bis 18 C-Atomen ist, oder ein mit einer monofunktionellen Carbonsäure endständig veresterter Polyoxyalkylenrest der Formel (5c), 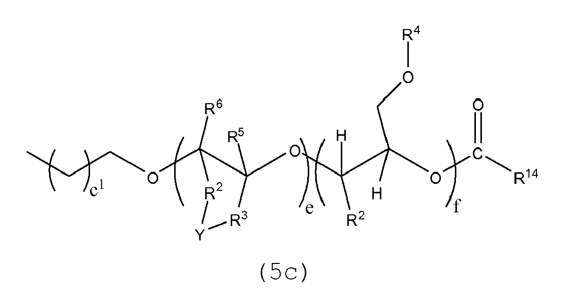 wobei
R¹⁴ ein gesättigter oder ein ein- oder mehrfach ungesättigter, entweder linearer oder verzweigter, aliphatischer oder aromatischer Kohlenwasserstoffrest mit 1-30 Kohlenstoffatomen ist, der seinerseits OH-Gruppen tragen kann, ist,
X⁴ entweder X¹ oder dem Fragment der Formel (5d) entspricht 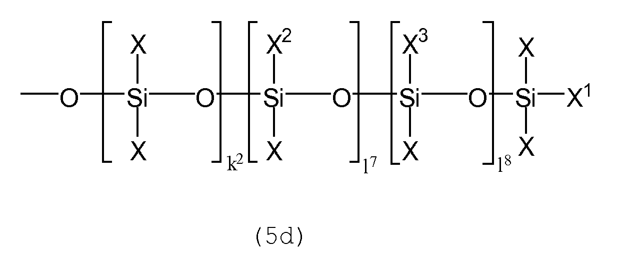 wobei
k, k¹ und k² unabhängig voneinander ganze Zahlen von 0 bis 500 sind,
l³, l⁴, l⁵, l⁶, l⁷ und l⁸ unabhängig voneinander ganze Zahlen von 0 bis 60 sind,
o eine ganze Zahl von 0 bis 10 ist, mit der Maßgabe, dass
X¹ mindestens einmal gleich X² ist, falls die Summe aus l³, l⁵ und l⁷ Null ist, und dass die Summe aus l³, l⁵ und l⁷ mindestens 1 ist, wenn X¹ ungleich X² ist,
wobei
a eine ganze Zahl von 1 bis 3 ist,
b eine ganze Zahl von 0 bis 2 ist,
die Summe von a und b gleich 3 ist,
c eine ganze Zahl von 0 bis 22 ist,
c¹ eine ganze Zahl von 0 bis 24 ist,
d eine ganze Zahl von 1 bis 500 ist,
e eine ganze Zahl von 0 bis 5000 ist,
n eine ganze Zahl von 2 bis 8 ist und
f, g, h, i und j jeweils ganze Zahlen von 0 bis 500 sind,
mit der Maßgabe, dass die Fragmente mit den Indices d bis j untereinander frei permutierbar, in der Sequenz innerhalb der Polyetherkette gegeneinander austauschbar sind und wobei die verschiedenen Monomereinheiten der Fragmente mit den Indexzahlen d bis j untereinander blockweise aufgebaut sein oder aber auch einer statistischen Verteilung unterliegen können
und mit der Maßgabe, dass die Fragmente mit den Indices k, k¹, k², l³, l⁴, l⁵, l⁶, l⁷, l⁸ und o untereinander frei permutierbar, innerhalb der Siloxankette gegeneinander austauschbar sind und wahlweise statistisch verteilt oder blockartig aneinandergereiht vorliegen können.

5. Verwendung nach Anspruch 4, **dadurch gekennzeichnet, dass** lineare Polyether-Siloxan-Polyether-Triblockcopolymere der Formel (6) enthalten sind, bei denen die mit Alkoxysilylgruppen ausgestatteten Polyetherketten über eine Si-O-C-Verknüpfung, b, an den Siloxankörper gebunden sind, 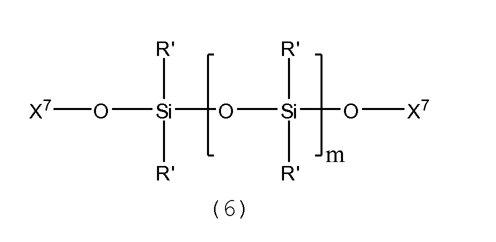 wobei
R' einem oder mehreren gleichen oder verschiedenen linearen oder verzweigten, gesättigten, einfach oder mehrfach ungesättigten Alkylresten mit 1 bis 20 Kohlenstoffatomen entspricht,
und
m eine ganze Zahl von 0 bis 5000 ist, und
X⁷ dem Polyetherfragment der Formel (6a) entspricht, 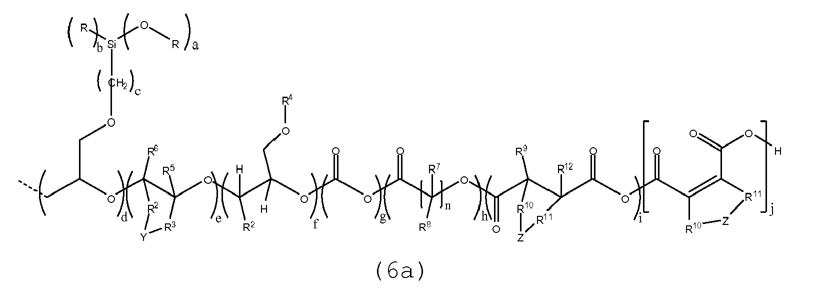 wobei die Substituenten R, R²-R¹², die Reste Y und Z sowie die Indizes a, b, c, d, e, f, g, h, i, j und n den zuvor für die Verbindungen der Formel (5a) genannten Definitionen entsprechen.

6. Verwendung gemäß zumindest einem der Ansprüche 1 bis 5, **dadurch gekennzeichnet, dass** neben dem Silylpolyether der Formel (1), (5) und/oder (6) weitere monomere Silane oder α,ω-silylterminierten Präpolymere eingesetzt werden.

7. Zusammensetzung zur Oberflächenmodifizierung oder Beschichtung enthaltend zumindest eine Verbindung der Formel (1), (5) und/oder (6).

8. Zusammensetzungen, die neben den Silylpolyethern 1 (1) auch andere Silylverbindungen, insbesondere solche der Formel (2),
AₓSiB₍₄₋ₓ₎ (2)
wobei A gleiche oder verschiedene nicht hydrolysierbare Gruppen, B = gleiche oder verschiedene hydrolysierbare Gruppen oder Hydroxygruppen und x = 1, 2, 3 oder 4 darstellt, und/oder als weitere Komponenten monomere, oligomere oder polymere Silane oder reaktive Silylgruppen tragende Komponenten wie ausgewählt aus der Gruppe umfassend Acrylate, Epoxide, Isocyanate, Carboxylate, Hydroxide, Lactone, Lactame, enthalten.

9. Zusammensetzung nach Anspruch 8, wobei für die Silylpolyether 1 der Formel (1) d > 1 gilt.

10. Verfahren zur Oberflächenmodifizierung von Partikeln oder Flächengebilden, **dadurch gekennzeichnet, dass** die Zusammensetzungen enthaltend Präpolymere der Formel (1) unter Vermischung und/oder in Gegenwart geeigneter Vernetzungskatalysatoren rein oder aus geeigneten organischen und/oder anorganischen Lösemitteln auf die Partikeloberflächen oder aus Emulsionen aufgebracht werden, wo sie dann unter kovalenter oder physikalischer Anbindung ausreagieren können.

11. Verfahren zur Oberflächenmodifizierung von Partikeloberflächen, **dadurch gekennzeichnet, dass** die Partikel oder die in einer Matrix dispergierten Partikel, durch Zugabe der Silylpolyether unter Durchmischung und optional Zugabe eines geeigneten Katalysators modifiziert werden.

12. Verfahren nach zumindest einem der Ansprüche 10 oder 11, **dadurch gekennzeichnet, dass** als zu modifizierende Oberflächen anorganische und/oder organische Partikel und/oder organomodifizierte Partikel und/oder deren Mischungen miteinander verwendet werden.

13. Reaktivverdünner, Emulsionen, Netzmittel, Lacke, Haftvermittler, Weichmacher, Thixotropiermittel, Fungizide, Flammschutzmittel, Pigmente, Füllstoffe, polymere Schäumen, organische Harzen oder Silikonharze, Melt-Flow-Index-Verbesserer Bauten, Textilien oder Fasern Gleitadditive, Schmiermittel, Mattierungsmittel, Ad- oder Absorbentien selbstdispergierbare Partikel partikuläre Emulgatoren Entschäumer, Bautenschutzmittel, Verkapselungsmittel, in Versiegelungen, antistatische Additive, Rieselhilfsmittel, mikrobizide Additive, Fluoreszenzmarker, Effektpigmente, Trennmittel, tieftemperaturbeständige Kabelbeschichtungen, leitfähige Beschichtungen, Leiterbahnen, antistatische Beschichtungen, elektronische und/oder elektrische Bauteile, Gummiteile und Membranen, Schlichte in der Textil- und Glasfaserindustrie, Papier, Additive für Toner, Abrasiva oder Faltenkaschierungsmittel in Kosmetika, Formulierungsmittel oder Trägermaterialien, Farb- und Konservierungsstoffe, Beschichtungen, Korrosionsschutzmittel, Farben, tribologische und/oder haptische Beschichtungen beschichtet und/oder modifiziert mit einer Zusammensetzung nach einem der Ansprüche 7 bis 9.

14. Verwendung der gemäß Anspruch 13 oberflächenmodifiziert ausgerüsteten Flächengebilde, Partikel und/oder Fasern im Hygiene-, Medizin-, Kosmetik-, Bau-, Automobil-, Heimtextil-, Bekleidungstextil-, Sport- und/oder Agrarbereich.

15. Verwendung der Silylpolyether-Zusammensetzungen gemäß zumindest einem der Ansprüche 13 oder 14 in kosmetischen Anwendungen als Additive in Lack- oder Nagellackformulierungen, zur Bautenhydrophobierung, als Dichtungsmassen und/oder Klebemassen, flammhemmende Massen, Kabelmäntel, Verbundwerkstoffe, Pulverlackhärter und/oder Flüssigpasten.
